# EUROPEAN PATENT APPLICATION

(11) **EP 3 222 288 A1**
(43) Date of publication of application: **27.09.2017**
(21) Application number: 16194601.7
(22) Date of filing: 26.07.2013
(51) Int. Cl.: A61K 38/51, A61K 48/00, A61K 35/741, A61K 35/747

(54) **GENETICALLY MODIFIED PROBIOTIC FOR THE TREATMENT OF PHENYLKETONURIA (PKU) DISEASE**

(30) Priority: 27.07.2012 US 201261676461 P
(62) Divisional of application: 13745543.2
(71) Applicant: University of North Texas, Denton, Texas 76207 (US)
(72) Inventor: Deming, Katherine, Irving, Texas 75061 (US); Allen, Michael, Denton, Texas 76209 (US); Von Herbing, Ione, Hunt, Denton, Texas 76210 (US)
(74) Representative: Tomkins & Co

(57) **Abstract**

A GMP adapted to provide the PAL gene for the treatment of PKU when administered orally. The GMP of the present invention may include a probiotic, a PAL gene to be expressed using the probiotic, wherein the PAL gene is functionally attached to a promoter and a ribosome binding site, and may be codon-optimized for expression in a certain host organism. A method of treating the metabolic disease of PKU by oral administration and ingestion of a GMP is also provided.

SEQUENCE LISTING

<110>  Deming, Katherine

<120>  GENETICALLY MODIFIED PROBIOTIC

<130>  UNTD-0075 PRO

<160>  15

<170>  PatentIn version 3.5

<210>  1
<211>  14
<212>  DNA
<213>  Artificial Sequence

<220>
<223>  Sequence to be added to to front of codon optimized AvPAL
       sequence.

<400>  1
aggagtaggt ataa                                                          14


<210>  2
<211>  56
<212>  DNA
<213>  Artificial Sequence

<220>
<223>  Sequence to be added to the end of AvPAL sequence.

<400>  2
tgataagtta aggggtgcat aaactgcatc ccttaactta tcaaaaaaaa gtcgac            56


<210>  3
<211>  1777
<212>  DNA
<213>  Artificial Sequence

<220>
<223>  Synthesized AvPAL 5' to 3'

<400>  3
aggagtaggt ataaatgaag actttatctc aagcacaatc taagactagt tctcaacaat      60

tctcatttac tggaaactca agtgctaatg ttatcattgg taatcaaaag cttactatta     120

acgatgttgc acgggttgca cgcaatggta ctttagttag tttaactaac aacactgaca     180

ttttacaagg cattcaagct agttgcgact atatcaataa cgctgttgaa tcaggagaac     240

caatttatgg tgttacttca ggattcggtg gaatggcaaa cgtcgctatt agtcgtgaac     300

aagcttctga acttcaaact aatcttgttt ggtttcttaa aacaggagct ggtaataaac     360

ttccattagc agatgttcgt gctgctatgt tattacgtgc aaattcacac atgcgtggcg     420

cttctggtat tcgtcttgaa ttaattaagc gtatggaaat tttccttaac gccggtgtta     480

caccttatgt ttacgaattc ggatcaattg gtgcaagtgg agatcttgtt cctttatctt     540

atattacagg ttctttaatt ggtttggatc catcattcaa agttgatttt aatggtaaag 600

aaatggatgc cccaacagct cttcgacaat taaatttatc accacttaca ttacttccaa 660

aagaaggtct tgctatgatg aatggtacat cagttatgac gggaattgct gctaattgcg 720

tttatgatac tcaaattctt actgctattg ccatgggtgt tcatgcctta gatattcaag 780

cacttaatgg cacaaatcaa agtttccacc cattcattca caattctaaa cctcacccag 840

gtcaactttg ggctgctgat caaatgattt ctcttttagc taatagtcaa cttgttcgcg 900

acgaattaga tggtaagcat gattatcgtg atcatgaact tattcaagac cgctatagtt 960

tacgttgctt accacaatat ttgggtccta ttgttgatgg aatttcacaa attgctaagc 1020

aaattgaaat cgaaattaat agtgtcactg acaatccatt aattgacgtt gataaccaag 1080

cctcatacca tggcggtaac tttttgggtc aatatgttgg catgggcatg gaccacttac 1140

gttactacat tggtcttctt gcaaagcatt tggatgtcca aattgcactt ttggccagtc 1200

cagaattcag taacggactt ccaccatcat tattgggaaa ccgggaacga aaggtaaaca 1260

tgggtcttaa gggtttgcaa atttgtggca actcaattat gccattgttg actttttacg 1320

gaaattcaat tgctgatcgc tttcctacac acgccgaaca atttaaccaa aatattaact 1380

ctcaaggata cacttctgct acgttagcac gacggtctgt tgatattttt caaaactacg 1440

tagctattgc attaatgttt ggagtacaag ctgttgattt acggacttac aaaaagacgg 1500

gccattatga tgctcgtgct tgtttaagtc cagctacaga acgtctttat tcagcagtac 1560

gtcatgttgt cggccaaaaa cctacatcag atcggccata tatttggaac gataacgaac 1620

aaggacttga cgaacatatt gctcgtattt ctgctgatat cgctgctggt ggtgtaattg 1680

ttcaagccgt acaagatatc cttccttgtt tacactaata atgataagtt aaggggtgca 1740

taaactgcat cccttaactt atcaaaaaaa agtcgac 1777

<210> 4
<211> 186
<212> DNA
<213> Artificial Sequence

<220>
<223> Synthetic ErmB promoter sequence

<400> 4
gcatgctagc tatagttcta gaggatacct ggttgattaa cgttagcctg gctacgtata 60

ctcctggaag tattaataga cgacctagga tgcatatgtt caagagtgtg ttgatagtgc 120

agtatcttaa aattttgtat aataggaatt gaagttaaat tagatgctaa aaatttgtaa 180

ttaaga 186

<210> 5

<211>  129
<212>  DNA
<213>  Artificial Sequence

<220>
<223>  Secretion tag sequence

<400>  5
atgtatactg aaaatacagg taaacatcac cgtaacggac ttcctgtgtg gctattacca      60

ttattggtcg ttattagttt ttggggcgta agccaaaata ttatggttgt tgatgcttct     120

tcagttgat                                                             129


<210>  6
<211>  2092
<212>  DNA
<213>  Artificial Sequence

<220>
<223>  Full secFuzErmAvPAL sequence

<400>  6
gcatgctagc tatagttcta gaggatacct ggttgattaa cgttagcctg gctacgtata      60

ctcctggaag tattaataga cgacctagga tgcatatgtt caagagtgtg ttgatagtgc     120

agtatcttaa aattttgtat aataggaatt gaagttaaat tagatgctaa aaatttgtaa     180

ttaagaagga gtaggtataa atgtatactg aaaatacagg taaacatcac cgtaacggac     240

ttcctgtgtg gctattacca ttattggtcg ttattagttt ttggggcgta agccaaaata     300

ttatggttgt tgatgcttct tcagttgata tgaagacttt atctcaagca caatctaaga     360

ctagttctca acaattctca tttactggaa actcaagtgc taatgttatc attggtaatc     420

aaaagcttac tattaacgat gttgcacggg ttgcacgcaa tggtacttta gttagtttaa     480

ctaacaacac tgacatttta caaggcattc aagctagttg cgactatatc aataacgctg     540

ttgaatcagg agaaccaatt tatggtgtta cttcaggatt cggtggaatg gcaaacgtcg     600

ctattagtcg tgaacaagct tctgaacttc aaactaatct tgtttggttt cttaaaacag     660

gagctggtaa taaacttcca ttagcagatg ttcgtgctgc tatgttatta cgtgcaaatt     720

cacacatgcg tggcgcttct ggtattcgtc ttgaattaat taagcgtatg gaaattttcc     780

ttaacgccgg tgttacacct tatgtttacg aattcggatc aattggtgca agtggagatc     840

ttgttccttt atcttatatt acaggttctt taattggttt ggatccatca ttcaaagttg     900

attttaatgg taaagaaatg gatgccccaa cagctcttcg acaattaaat ttatcaccac     960

ttacattact tccaaaagaa ggtcttgcta tgatgaatgg tacatcagtt atgacgggaa    1020

ttgctgctaa ttgcgtttat gatactcaaa ttcttactgc tattgccatg ggtgttcatg    1080

ccttagatat tcaagcactt aatggcacaa atcaaagttt ccacccattc attcacaatt    1140

ctaaacctca cccaggtcaa ctttgggctg ctgatcaaat gatttctctt ttagctaata    1200

gtcaacttgt tcgcgacgaa ttagatggta agcatgatta tcgtgatcat gaacttattc 1260

aagaccgcta tagtttacgt tgcttaccac aatatttggg tcctattgtt gatggaattt 1320

cacaaattgc taagcaaatt gaaatcgaaa ttaatagtgt cactgacaat ccattaattg 1380

acgttgataa ccaagcctca taccatggcg gtaacttttt gggtcaatat gttggcatgg 1440

gcatggacca cttacgttac tacattggtc ttcttgcaaa gcatttggat gtccaaattg 1500

cacttttggc cagtccagaa ttcagtaacg gacttccacc atcattattg ggaaaccggg 1560

aacgaaaggt aaacatgggt cttaagggtt tgcaaatttg tggcaactca attatgccat 1620

tgttgacttt ttacggaaat tcaattgctg atcgctttcc tacacacgcc gaacaattta 1680

accaaaatat taactctcaa ggatacactt ctgctacgtt agcacgacgg tctgttgata 1740

tttttcaaaa ctacgtagct attgcattaa tgtttggagt acaagctgtt gatttacgga 1800

cttacaaaaa gacgggccat tatgatgctc gtgcttgttt aagtccagct acagaacgtc 1860

tttattcagc agtacgtcat gttgtcggcc aaaaacctac atcagatcgg ccatatattt 1920

ggaacgataa cgaacaagga cttgacgaac atattgctcg tatttctgct gatatcgctg 1980

ctggtggtgt aattgttcaa gccgtacaag atatccttcc ttgtttacac taataatgat 2040

aagttaaggg gtgcataaac tgcatccctt aacttatcaa aaaaaagtcg ac 2092

<210> 7
<211> 7068
<212> DNA
<213> Artificial Sequence

<220>
<223> pSLERGT sequence

<400> 7
tcttccgctt cctcgctcac tgactcgctg cgctcggtcg ttcggctgcg gcgagcggta 60

tcagctcact caaaggcggt aatacggtta tccacagaat caggggataa cgcaggaaag 120

aacatgtgag caaaaggcca gcaaaaggcc aggaaccgta aaaaggccgc gttgctggcg 180

tttttccata ggctccgccc ccctgacgag catcacaaaa atcgacgctc aagtcagagg 240

tggcgaaacc cgacaggact ataaagatac caggcgtttc cccctggaag ctccctcgtg 300

cgctctcctg ttccgaccct gccgcttacc ggatacctgt ccgcctttct cccttcggga 360

agcgtggcgc tttctcatag ctcacgctgt aggtatctca gttcggtgta ggtcgttcgc 420

tccaagctgg gctgtgtgca cgaacccccc gttcagcccg accgctgcgc cttatccggt 480

aactatcgtc ttgagtccaa cccggtaaga cacgacttat cgccactggc agcagccact 540

ggtaacagga ttagcagagc gaggtatgta ggcggtgcta cagagttctt gaagtggtgg 600

cctaactacg gctacactag aagaacagta tttggtatct gcgctctgct gaagccagtt 660

accttcggaa aaagagttgg tagctcttga tccggcaaac aaaccaccgc tggtagcggt 720

ggtttttttg tttgcaagca gcagattacg cgcagaaaaa aaggatctca agaagatcct 780

ttgatctttt ctacggggtc tgacgctcag tggaacgaaa actcacgtta agggattttg 840

gtcatgagat tatcaaaaag gatcttcacc tagatccttt taaattaaaa atgaagtttt 900

aaatcaatct aaagtatata tgagtaaact tggtctgaca gttaccaatg cttaatcagt 960

gaggcaccta tctcagcgat ctgtctattt cgttcatcca tagttgcctg actccccgtc 1020

gtgtagataa ctacgatacg ggagggctta ccatctggcc ccagtgctgc aatgataccg 1080

cgagacccac gctcaccggc tccagattta tcagcaataa accagccagc cggaagggcc 1140

gagcgcagaa gtggtcctgc aactttatcc gcctccatcc agtctattaa ttgttgccgg 1200

gaagctagag taagtagttc gccagttaat agtttgcgca acgttgttgc cattgctaca 1260

ggcatcgtgg tgtcacgctc gtcgtttggt atggcttcat tcagctccgg ttcccaacga 1320

tcaaggcgag ttacatgatc ccccatgttg tgcaaaaaag cggttagctc cttcggtcct 1380

ccgatcgttg tcagaagtaa gttggccgca gtgttatcac tcatggttat ggcagcactg 1440

cataattctc ttactgtcat gccatccgta agatgctttt ctgtgactgg tgagtactca 1500

accaagtcat tctgagaata gtgtatgcgg cgaccgagtt gctcttgccc ggcgtcaata 1560

cgggataata ccgcgccaca tagcagaact ttaaaagtgc tcatcattgg aaaacgttct 1620

tcggggcgaa aactctcaag gatcttaccg ctgttgagat ccagttcgat gtaacccact 1680

cgtgcaccca actgatcttc agcatctttt actttcacca gcgtttctgg gtgagcaaaa 1740

acaggaaggc aaaatgccgc aaaaaaggga ataagggcga cacggaaatg ttgaatactc 1800

atactcttcc tttttcaata ttattgaagc atttatcagg gttattgtct catgagcgga 1860

tacatatttg aatgtattta gaaaaataaa caaatagggg ttccgcgcac atttccccga 1920

aaagtgccac ctgacgtcta agaaaccatt attatcatga cattaaccta taaaaatagg 1980

cgtatcacga ggccctttcg tctcgcgcgt ttcggtgatg acggtgaaaa cctctgacac 2040

atgcagctcc cggagacggt cacagcttgt ctgtaagcgg atgccgggag cagacaagcc 2100

cgtcagggcg cgtcagcggg tgttggcggg tgtcggggct ggcttaacta tgcggcatca 2160

gagcagattg tactgagagt gcaccataaa attgtaaacg ttaatatttt gttaaaattc 2220

gcgttaaatt tttgttaaat cagctcattt tttaaccaat aggccgaaat cggcaaaatc 2280

ccttataaat caaaagaata gcccgagata gggttgagtg ttgttccagt ttggaacaag 2340

agtccactat taaagaacgt ggactccaac gtcaaagggc gaaaaaccgt ctatcagggc 2400

gatggcccac tacgtgaacc atcacccaaa tcaagttttt tggggtcgag gtgccgtaaa 2460

gcactaaatc ggaaccctaa agggagcccc cgatttagag cttgacgggg aaagccggcg 2520

aacgtggcga gaaaggaagg gaagaaagcg aaaggagcgg gcgctagggc gctggcaagt 2580

gtagcggtca cgctgcgcgt aaccaccaca cccgccgcgc ttaatgcgcc gctacagggc 2640

gcgtactatg gttgctttga cgtatgcggt gtgaaatacc gcacagatgc gtaaggagaa 2700

aataccgcat caggcgccat tcgccattca ggctgcgcaa ctgttgggaa gggcgatcgg 2760

tgcgggcctc ttcgctatta cgccagctgg cgaaaggggg atgtgctgca aggcgattaa 2820

gttgggtaac gccagggttt tcccagtcac gacgttgtaa aacgacggcc agtgaattcg 2880

aatggccatg gaggatttat tctctccgca gttttgagct taatcgtctc attaactact 2940

gcacttttct ccgattggct tcgtcggaga aaataatcta acggcatgaa gcgcaaatta 3000

aaagccactc aactatgggc ctagttgggt ggcttttttg caaccattac ggttttactt 3060

ctctataaga attatagcat caatcttcat cattagccaa ctcaatagaa gctcgatata 3120

gatcatgttg agctttatcc aagctatcta aaagttcgcc ataaatatca atatttttct 3180

tttcagcaac caattttttc aaatcttgaa gattatcagc aatctgttct gccataactc 3240

catagatttc agcttgtttc ttcatattgt caattgtctt aatatcttgt aactccattc 3300

ctatcactcc catcttacat agttacttac cttagaattc caacgatata tcacagtatc 3360

tacaacatca tctttttctt tttcgtcatc cacattaatt agatcatctt ctttttcgtc 3420

cagcaataac tcatgatgaa tctcttttag taatcccgca aagcttaatt gtcgagttcc 3480

agccagagca tgctcgagct cgtcaacaac aactaaatcg cctttctcat catccgtaat 3540

ataatcataa tctttaactt gatatttact tacttctttt gccgaagcaa tcaaactatt 3600

atcttttttt gacgcccgaa ttttcttgat attaacaatc ggcttgtagt ctaatttcct 3660

tgctcgcttc cataattgtg accaattctc ttgggtaaga taatgaccct tagaaaaata 3720

actagatttc accattaaca aaatatgtac atgttgatgg aaagttagac tatctcgatt 3780

gaccgttatt tctgtagatc gcacataacc aagaagatct ttttttacat ctttatattg 3840

aaaaagccga taaattgaac gattcatcga agtaagattt tttctaagct cgcccaactt 3900

tgagctttcc tcagttaatg tcaagaacag aaatatagca ctaggatcct ttttatatgc 3960

tgtatctaaa atttgacgca actcataaga atttttcatc gatcggcgcc agctacataa 4020

tggacaaagt ctcgaatgac aaaaccacgt ttgatataac tttaaaccat tatcagtttt 4080

cgcaaattga agaacttctc cgcactttga aacgttatga gcttttttaa aatttaaaac 4140

ctccagatat tgggaataag ttaagttagc aagttttttc tgcttccacg gtctaacttt 4200

cccattacta gaggtatctt ttagaatttc ttttttatac atttcttata cctctaactg 4260

caaaaactac aagcagtcag agttagttat cattgatata ttcctaatga catattataa 4320

tagggttcga agttaataac gataactaac tcatgcatcg acggccaaat cgaacaactg 4380

catgagtttt ttatttaatt cttacaatgc ttattataca ccaaaaagtg ttatatttta 4440

aaatcctaat aaacccagtc atatcaaggg tttaacccaa tttcaaaaaa tggcgttgtt 4500

tctatatgta tcaagataag aagaaactcg cttacagcga gtttctaaat aggggtttca 4560

cccctaaaac cctgaccagc tgagccagct ggaccgcatt tcccctcgcc gggcgggcgt 4620

aaagcggaaa aaatgacttt tttctcgctt tcgctcaact agggtcttcc cgtctcacca 4680

tgtcaagcgc tgcccttccg ctacgctcca ggcgtttgtc gcttgacact cacctactaa 4740

cagcgggctt cgccaccagt ctactatctg caacacggca acaagattaa tcagaatatg 4800

cattgaagca gtgcgctccg ctccttgtct tgcgacaaag cgagcgcact gcttcaatgc 4860

ttttttattt tctgaattcc ataactcgat aattagaaaa agttagtccg ttagaaacgt 4920

ctgccacgcc gaggaagtca ttccagtttg accactcaaa tcatcagcga catcattgag 4980

ataatcaatc agcaattttc tagtttctat ctgctttgga atttttacca cagccaaaat 5040

tgagtcccac ttagcaatca caaaagattt tctgacggct ttattagcct tttccgcact 5100

aatatttttc gctttatctc gtttattttg atcggtcgat ggtatccgat cgactgttgt 5160

tacagcattt gacgcaggtt gctagataaa cgactagacc gaataatttt tattccaact 5220

atttttgcaa ccaaaaaaat cacaaaataa tcaaccaata aaacgataac taacttcttg 5280

ctacatccga ttaatttttc tccagcctgc cagaaatcgg cccaaatatt gaaatattga 5340

ggaaattttg ggctgaaaaa aacattaatt ttttcggcaa acaagccaac acagccaagc 5400

agaaaagcta agaaaataag ttccagccaa ttaaaaaatc gccacgaaag agaaaaaaat 5460

cggtcaaaat aaatcgaact tgatttcatt ttctagcccc cgaagtcttc gataaacggc 5520

gccataaaag gaactacacg gttttccggt tgagtacttg caagccctaa ccccttagaa 5580

aagccgtcat gattaacaac tgttgtcgac tcagctaggg ccctatatat ggatccaatt 5640

gcaatgatca tcatgacaga tctgcgcgcg atcgatatca gcgctttaaa tttgcgcatg 5700

ctagctatag ttctagaggt accggttgtt aacgttagcc ggctacgtat actccggaat 5760

attaataggc ctaggatgca tatgcttaga agcaaactta agagtgtgtt gatagtgcag 5820

tatcttaaaa ttttgtataa taggaattga agttaaatta gatgctaaaa atttgtaatt 5880

aagaaggagt gattacatga acaaaaatat aaaatattct caaaactttt taacgagtga 5940

aaaagtactc aaccaaataa taaaacaatt gaatttaaaa gaaaccgata ccgtttacga 6000

aattggaaca ggtaaagggc atttaacgac gaaactggct aaaataagta aacaggtaac 6060

gtctattgaa ttagacagtc atctattcaa cttatcgtca gaaaaattaa aactgaatac 6120

tcgtgtcact ttaattcacc aagatattct acagtttcaa ttccctaaca aacagaggta 6180

taaaattgtt gggagtattc cttaccattt aagcacacaa attattaaaa aagtggtttt 6240

tgaaagccat gcgtctgaca tctatctgat tgttgaagaa ggattctaca agcgtacctt 6300

ggatattcac cgaacactag ggttgctctt gcacactcaa gtctcgattc agcaattgct 6360

taagctgcca gcggaatgct ttcatcctaa accaaaagta aacagtgtct taataaaact 6420

tacccgccat accacagatg ttccagataa atattggaag ctatatacgt actttgtttc 6480

aaaatgggtc aatcgagaat atcgtcaact gtttactaaa aatcagtttc atcaagcaat 6540

gaaacacgcc aaagtaaaca atttaagtac cgttacttat gagcaagtat tgtctatttt 6600

taatagttat ctattattta acgggaggaa ataattctat gagtcgcttt tgtaaatttg 6660

gaaagttaca cgttactaaa gggaatgtag ataaattatt aggtatacta ctgacagctt 6720

ccaagcatat ggcggccgcc tgcagctggc gccatcgata cgcgtacgtc gcgaccgcgg 6780

acatgtacag agctcgagaa gtactagtgg ccacgtgggc cgtgcacctt aagcttggcg 6840

taatcatggt catagctgtt tcctgtgtga aattgttatc cgctcacaat tccacacaac 6900

atacgagccg gaagcataaa gtgtaaagcc tggggtgcct aatgagtgag ctaactcaca 6960

ttaattgcgt tgcgctcact gcccgctttc cagtcgggaa acctgtcgtg ccagctgcat 7020

taatgaatcg gccaacgcgc ggggagaggc ggtttgcgta ttgggcgc 7068

<210> 8
<211> 2764
<212> DNA
<213> Artificial Sequence

<220>
<223> Predicted pGT232 from PCR of pNCKH103

<400> 8
tagctgagtc gacaacagtt gttaatcatg acggcttttc taaggggtta gggcttcaag 60

tactcaaccg gaaaaccgtg tagttccttt tatggcgccg tttatcgaag acttcggggg 120

ctagaaaatg aaatcaagtt cgatttattt tgaccgattt ttttctcttt cgtggcgatt 180

ttttaattgg ctggaactta ttttcttagc ttttctgctt ggctgtgttg gcttgtttgc 240

cgaaaaaatt aatgtttttt tcagcccaaa atttcctcaa tatttcaata tttgggccga 300

tttctggcag gctggagaaa aattaatcgg atgtagcaag aagttagtta tcgttttatt 360

ggttgattat tttgtgattt ttttggttgc aaaaatagtt ggaataaaaa ttattcggtc 420

tagtcgttta tctagcaacc tgcgtcaaat gctgttaaca acagtcgatc ggataccatc 480

gaccgatcaa aataaacgag ataaagcgaa aaatattagt gcggaaaagg ctaataaagc 540

cgtcagaaaa tcttttgtga ttgctaagtg ggactcaatt ttggctgtgg taaaaattcc 600

aaagcagata gaaactagaa aattgctgat tgattatctc aatgatgtcg ctgatgattt 660

gagtggtcaa actggaatga cttcctcggc gtggcagacg tttctaacgg actaactttt 720

tctaattatc gagttatgga attcagaaaa taaaaaagca ttgaagcagt gcgctcgctt 780

tgtcgcaaga caaggagcgg agcgcactgc ttcaatgcat attctgatta atcttgttgc 840

cgtgttgcag atagtagact ggtgcgaagc ccgctgttag taggtgagtg tcaagcgaca 900

caacgcctgg agcgtgagcg gaagggcagc gcttgacatg gtgagacggg aagaccctag 960

ttgagcgaaa gcgagaaaaa agtcattttt tccgctttag cccgcccggc gaggggaaat 1020

gcggtccagc tgggtcaggg ttttaggggg tgaaacatcc ttagaaactc gctgtaagcg 1080

agtttcttct tatcttgata catatagaaa caacgccatt ttttgaaatt gggttaaacc 1140

cttgatatga ctgggtttat taggatttta aaatataaca ctttttggtg tataataagc 1200

attgtaagaa ttaaataaaa aactcatgca gttgttcgat ttggccgtcg atgcatcagt 1260

tagttatcgt tattaacttc gaaccctatt ataatatgtc attaggaata tatcaatgat 1320

aactaactct gactgcttgt agtttttgca gttagaggta taagaaatgt ataaaaaaga 1380

aattctaaaa gatacctcta gtaatgggaa agttagaccg tggaagcaga aaaaacttgc 1440

taacttaact tattcccaat atctggaggt tttaaatttt aaaaaagctc ataacgtttc 1500

aaagtgcgga gaagttcttc aatttgcgaa aactgataat ggtttaaagt tatatcaaac 1560

gtggttttgt cattcgagac tttgtccatt atgtagctgg cgccgatcga tgaaaaattc 1620

ttatgagttg cgtcaaattt tagatacagc atataaaaag gatcctagtg ctatatttct 1680

cttcttgaca ttaactgagg aaagctcaaa gttgggcgag cttagaaaaa atcttacttc 1740

gatgaatcgt tcaatttatc ggctttttca atataaagat gtaaaaaaag atcttcttgg 1800

ttatgtgcga tctacagaaa taacggtcaa tcgagatagt ctaactttcc atcaacatgt 1860

acatattttg ttaatggtga aatctagtta tttttctaag ggtcattatc ttacccaaga 1920

gaattggtca caattatgga agcgagcaag gaaattagac tacaagccga ttgttaatat 1980

caagaaaatt cgggcgtcaa aaaaagataa tagtttgatt gcttcggcaa aagaagtaag 2040

taaatatcaa gttaaagatt atgattatat tacggatgat gagaaaggcg atttagttgt 2100

tgttgacgag ctcgagcatg ctctggctgg aactcgacaa ttaagctttg cgggattact 2160

aaaagagatt catcatgagt tattgctgga cgaaaaagaa gatgatctaa ttaatgtgga 2220

tgacgaaaaa gaaaaagatg atgttgtaga tactgtgata tatcgttgga attctaaggt 2280

aagtaactat gtaagatggg agtgatagga atggagttac aagatattaa gacaattgac 2340

aatatgaaga aacaagctga aatctatgga gttatgccag aacagattgc tgataatctt 2400

caagatttga aaaaattggt tgctgaaaag aaaaatattg atatttatgg cgaactttta 2460

gatagcttgg ataaagctca acatgatcta tatcgagctt ctattgagtt ggctaatgat 2520

gaagattgat gctataattc ttatagagaa gtaaaaccgt aatggttgca aaaaagccac 2580

ccaactaggc ccatagttga gtggctttta atttgcgctt catgccgtta gattattttc 2640

tccgacgaag ccaatcggag aaaagtgcag tagttaatga gacgattaag ctcaaaactg 2700

cggagagaat aaatcctcca tggtttctta gacggagaga ataaatcctc catggtttct 2760

taga                                                                  2764

<210>  9
<211>  4356
<212>  DNA
<213>  Artificial Sequence

<220>
<223>  pSLER1 sequence

<400>  9
tcttccgctt cctcgctcac tgactcgctg cgctcggtcg ttcggctgcg gcgagcggta      60

tcagctcact caaaggcggt aatacggtta tccacagaat caggggataa cgcaggaaag     120

aacatgtgag caaaaggcca gcaaaaggcc aggaaccgta aaaaggccgc gttgctggcg     180

tttttccata ggctccgccc ccctgacgag catcacaaaa atcgacgctc aagtcagagg     240

tggcgaaacc cgacaggact ataaagatac caggcgtttc cccctggaag ctccctcgtg     300

cgctctcctg ttccgaccct gccgcttacc ggatacctgt ccgcctttct cccttcggga     360

agcgtggcgc tttctcatag ctcacgctgt aggtatctca gttcggtgta ggtcgttcgc     420

tccaagctgg gctgtgtgca cgaacccccc gttcagcccg accgctgcgc cttatccggt     480

aactatcgtc ttgagtccaa cccggtaaga cacgacttat cgccactggc agcagccact     540

ggtaacagga ttagcagagc gaggtatgta ggcggtgcta cagagttctt gaagtggtgg     600

cctaactacg gctacactag aagaacagta tttggtatct gcgctctgct gaagccagtt     660

accttcggaa aaagagttgg tagctcttga tccggcaaac aaaccaccgc tggtagcggt     720

ggtttttttg tttgcaagca gcagattacg cgcagaaaaa aaggatctca agaagatcct     780

ttgatctttt ctacggggtc tgacgctcag tggaacgaaa actcacgtta agggattttg     840

gtcatgagat tatcaaaaag gatcttcacc tagatccttt taaattaaaa atgaagtttt     900

aaatcaatct aaagtatata tgagtaaact tggtctgaca gttaccaatg cttaatcagt     960

gaggcaccta tctcagcgat ctgtctattt cgttcatcca tagttgcctg actccccgtc    1020

gtgtagataa ctacgatacg ggagggctta ccatctggcc ccagtgctgc aatgataccg    1080

cgagacccac gctcaccggc tccagattta tcagcaataa accagccagc cggaagggcc    1140

gagcgcagaa gtggtcctgc aactttatcc gcctccatcc agtctattaa ttgttgccgg    1200

gaagctagag taagtagttc gccagttaat agtttgcgca acgttgttgc cattgctaca    1260

ggcatcgtgg tgtcacgctc gtcgtttggt atggcttcat tcagctccgg ttcccaacga    1320

tcaaggcgag ttacatgatc ccccatgttg tgcaaaaaag cggttagctc cttcggtcct    1380

ccgatcgttg tcagaagtaa gttggccgca gtgttatcac tcatggttat ggcagcactg    1440

cataattctc ttactgtcat gccatccgta agatgctttt ctgtgactgg tgagtactca    1500

accaagtcat tctgagaata gtgtatgcgg cgaccgagtt gctcttgccc ggcgtcaata 1560
cgggataata ccgcgccaca tagcagaact ttaaaagtgc tcatcattgg aaaacgttct 1620
tcggggcgaa aactctcaag gatcttaccg ctgttgagat ccagttcgat gtaacccact 1680
cgtgcaccca actgatcttc agcatctttt actttcacca gcgtttctgg gtgagcaaaa 1740
acaggaaggc aaaatgccgc aaaaaaggga ataagggcga cacggaaatg ttgaatactc 1800
atactcttcc tttttcaata ttattgaagc atttatcagg gttattgtct catgagcgga 1860
tacatatttg aatgtattta gaaaaataaa caaatagggg ttccgcgcac atttccccga 1920
aaagtgccac ctgacgtcta agaaaccatt attatcatga cattaaccta taaaaatagg 1980
cgtatcacga ggccctttcg tctcgcgcgt ttcggtgatg acggtgaaaa cctctgacac 2040
atgcagctcc cggagacggt cacagcttgt ctgtaagcgg atgccgggag cagacaagcc 2100
cgtcagggcg cgtcagcggg tgttggcggg tgtcggggct ggcttaacta tgcggcatca 2160
gagcagattg tactgagagt gcaccataaa attgtaaacg ttaatatttt gttaaaattc 2220
gcgttaaatt tttgttaaat cagctcattt tttaaccaat aggccgaaat cggcaaaatc 2280
ccttataaat caaaagaata gcccgagata gggttgagtg ttgttccagt ttggaacaag 2340
agtccactat taaagaacgt ggactccaac gtcaaagggc gaaaaaccgt ctatcagggc 2400
gatggcccac tacgtgaacc atcacccaaa tcaagttttt tggggtcgag gtgccgtaaa 2460
gcactaaatc ggaaccctaa agggagcccc cgatttagag cttgacgggg aaagccggcg 2520
aacgtggcga gaaaggaagg gaagaaagcg aaaggagcgg gcgctagggc gctggcaagt 2580
gtagcggtca cgctgcgcgt aaccaccaca cccgccgcgc ttaatgcgcc gctacagggc 2640
gcgtactatg gttgctttga cgtatgcggt gtgaaatacc gcacagatgc gtaaggagaa 2700
aataccgcat caggcgccat tcgccattca ggctgcgcaa ctgttgggaa gggcgatcgg 2760
tgcgggcctc ttcgctatta cgccagctgg cgaaaggggg atgtgctgca aggcgattaa 2820
gttgggtaac gccagggttt tcccagtcac gacgttgtaa aacgacggcc agtgaattcg 2880
aatggccatg ggacgtcgac ctgaggtaat tataacccgg gccctatata tggatccaat 2940
tgcaatgatc atcatgacag atctgcgcgc gatcgatatc agcgctttaa atttgcgcat 3000
gctagctata gttctagagg taccggttgt taacgttagc cggctacgta tactccggaa 3060
tattaatagg cctaggatgc atatgttaag agtgtgttga tagtgcagta tcttaaaatt 3120
ttgtataata ggaattgaag ttaaattaga tgctaaaaat ttgtaattaa gaaggagtga 3180
ttacatgaac aaaaatataa aatattctca aaacttttta acgagtgaaa aagtactcaa 3240
ccaaataata aaacaattga atttaaaaga aaccgatacc gtttacgaaa ttggaacagg 3300
taaagggcat ttaacgacga aactggctaa aataagtaaa caggtaacgt ctattgaatt 3360
agacagtcat ctattcaact tatcgtcaga aaaattaaaa ctgaatactc gtgtcacttt 3420

aattcaccaa gatattctac agtttcaatt ccctaacaaa cagaggtata aaattgttgg 3480

gagtattcct taccatttaa gcacacaaat tattaaaaaa gtggtttttg aaagccatgc 3540

gtctgacatc tatctgattg ttgaagaagg attctacaag cgtaccttgg atattcaccg 3600

aacactaggg ttgctcttgc acactcaagt ctcgattcag caattgctta agctgccagc 3660

ggaatgcttt catcctaaac caaaagtaaa cagtgtctta ataaaactta cccgccatac 3720

cacagatgtt ccagataaat attggaagct atatacgtac tttgtttcaa aatgggtcaa 3780

tcgagaatat cgtcaactgt ttactaaaaa tcagtttcat caagcaatga aacacgccaa 3840

agtaaacaat ttaagtaccg ttacttatga gcaagtattg tctattttta atagttatct 3900

attatttaac gggaggaaat aattctatga gtcgcttttg taaatttgga aagttacacg 3960

ttactaaagg gaatgtagat aaattattag gtatactact gacagcttcc aagcatatgg 4020

cggccgcctg cagctggcgc catcgatacg cgtacgtcgc gaccgcggac atgtacagag 4080

ctcgagaagt actagtggcc acgtgggccg tgcaccttaa gcttggcgta atcatggtca 4140

tagctgtttc ctgtgtgaaa ttgttatccg ctcacaattc cacacaacat acgagccgga 4200

agcataaagt gtaaagcctg gggtgcctaa tgagtgagct aactcacatt aattgcgttg 4260

cgctcactgc ccgctttcca gtcgggaaac ctgtcgtgcc agctgcatta atgaatcggc 4320

caacgcgcgg ggagaggcgg tttgcgtatt gggcgc 4356

<210> 10
<211> 25
<212> DNA
<213> Artificial Sequence

<220>
<223> Forward primer pGT232 amplification reaction.

<400> 10
tagctgagtc gacaacagtt gttaa 25

<210> 11
<211> 30
<212> DNA
<213> Artificial Sequence

<220>
<223> Reverse primer pGT232 amplification reaction.

<400> 11
tctaagaaac catggaggat ttattctctc 30

<210> 12
<211> 3054
<212> DNA
<213> Artificial Sequence

<220>
<223>  Sequenced pGT232 inserted into pSLER1 for pSLERGT

<400>  12
ccatggagga tttattctct ccgcagtttt gagcttaatc gtctcattaa ctactgcact      60

tttctccgat tggcttcgtc ggagaaaata atctaacggc atgaagcgca aattaaaagc     120

cactcaacta tgggcctagt tgggtggctt ttttgcaacc attacggttt tacttctcta     180

taagaattat agcatcaatc ttcatcatta gccaactcaa tagaagctcg atatagatca     240

tgttgagctt tatccaagct atctaaaagt tcgccataaa tatcaatatt tttcttttca     300

gcaaccaatt ttttcaaatc ttgaagatta tcagcaatct gttctgccat aactccatag     360

atttcagctt gtttcttcat attgtcaatt gtcttaatat cttgtaactc cattcctatc     420

actcccatct tacatagtta cttaccttag aattccaacg atatatcaca gtatctacaa     480

catcatcttt ttctttttcg tcatccacat taattagatc atcttctttt tcgtccagca     540

ataactcatg atgaatctct tttagtaatc ccgcaaagct taattgtcga gttccagcca     600

gagcatgctc gagctcgtca acaacaacta aatcgccttt ctcatcatcc gtaatataat     660

cataatcttt aacttgatat ttacttactt cttttgccga agcaatcaaa ctattatctt     720

tttttgacgc ccgaattttc ttgatattaa caatcggctt gtagtctaat ttccttgctc     780

gcttccataa ttgtgaccaa ttctcttggg taagataatg acccttagaa aaataactag     840

atttcaccat taacaaaata tgtacatgtt gatggaaagt tagactatct cgattgaccg     900

ttatttctgt agatcgcaca taaccaagaa gatctttttt tacatcttta tattgaaaaa     960

gccgataaat tgaacgattc atcgaagtaa gattttttct aagctcgccc aactttgagc    1020

tttcctcagt taatgtcaag aacagaaata tagcactagg atccttttta tatgctgtat    1080

ctaaaatttg acgcaactca taagaatttt tcatcgatcg gcgccagcta cataatggac    1140

aaagtctcga atgacaaaac cacgtttgat ataactttaa accattatca gttttcgcaa    1200

attgaagaac ttctccgcac tttgaaacgt tatgagcttt tttaaaattt aaaacctcca    1260

gatattggga ataagttaag ttagcaagtt ttttctgctt ccacggtcta actttcccat    1320

tactagaggt atcttttaga atttcttttt tatacatttc ttatacctct aactgcaaaa    1380

actacaagca gtcagagtta gttatcattg atatattcct aatgacatat tataataggg    1440

ttcgaagtta ataacgataa ctaactcatg catcgacggc caaatcgaac aactgcatga    1500

gttttttatt taattcttac aatgcttatt atacaccaaa aagtgttata ttttaaaatc    1560

ctaataaacc cagtcatatc aagggtttaa cccaatttca aaaaatggcg ttgtttctat    1620

atgtatcaag ataagaagaa actcgcttac agcgagtttc taaatagggg tttcacccct    1680

aaaaccctga ccagctgagc cagctggacc gcatttcccc tcgccgggcg ggcgtaaagc    1740

ggaaaaaatg acttttttct cgctttcgct caactagggt cttcccgtct caccatgtca    1800

agcgctgccc ttccgctacg ctccaggcgt ttgtcgcttg acactcacct actaacagcg 1860

ggcttcgcca ccagtctact atctgcaaca cggcaacaag attaatcaga atatgcattg 1920

aagcagtgcg ctccgctcct tgtcttgcga caaagcgagc gcactgcttc aatgcttttt 1980

tattttctga attccataac tcgataatta gaaaaagtta gtccgttaga aacgtctgcc 2040

acgccgagga agtcattcca gtttgaccac tcaaatcatc agcgacatca ttgagataat 2100

caatcagcaa ttttctagtt tctatctgct ttggaatttt taccacagcc aaaattgagt 2160

cccacttagc aatcacaaaa gattttctga cggctttatt agccttttcc gcactaatat 2220

ttttcgcttt atctcgttta ttttgatcgg tcgatggtat ccgatcgact gttgttacag 2280

catttgacgc aggttgctag ataaacgact agaccgaata atttttattc caactatttt 2340

tgcaaccaaa aaaatcacaa aataatcaac caataaaacg ataactaact tcttgctaca 2400

tccgattaat ttttctccag cctgccagaa atcggcccaa atattgaaat attgaggaaa 2460

ttttgggctg aaaaaaacat taattttttc ggcaaacaag ccaacacagc caagcagaaa 2520

agctaagaaa ataagttcca gccaattaaa aaatcgccac gaaagagaaa aaaatcggtc 2580

aaaataaatc gaacttgatt tcattttcta gcccccgaag tcttcgataa acggcgccat 2640

aaaaggaact acacggtttt ccggttgagt acttgcaagc cctaacccct tagaaaagcc 2700

gtcatgatta acaactgttg tcgactcagc tagggcccta tatatggatc caattgcaat 2760

gatcatcatg acagatctgc gcgcgatcga tatcagcgct ttaaatttgc gcatgctagc 2820

tatagttcta gaggtaccgg ttgttaacgt tagccggcta cgtatactcc ggaatattaa 2880

taggcctagg atgcatatgc ttagaagcaa acttaagagt gtgttgatag tgcagtatct 2940

taaaattttg tataatagga attgaagtta aattagatgc taaaaatttg taattaagaa 3000

ggagtgatta catgaacaaa aatataaaat attctcaaaa ctttttaacg agtg 3054

<210> 13
<211> 9013
<212> DNA
<213> Artificial Sequence

<220>
<223> pHENOMM

<400> 13
tcttccgctt cctcgctcac tgactcgctg cgctcggtcg ttcggctgcg gcgagcggta 60

tcagctcact caaaggcggt aatacggtta tccacagaat caggggataa cgcaggaaag 120

aacatgtgag caaaaggcca gcaaaaggcc aggaaccgta aaaaggccgc gttgctggcg 180

tttttccata ggctccgccc ccctgacgag catcacaaaa atcgacgctc aagtcagagg 240

tggcgaaacc cgacaggact ataaagatac caggcgtttc cccctggaag ctccctcgtg 300

cgctctcctg ttccgaccct gccgcttacc ggatacctgt ccgcctttct cccttcggga 360

agcgtggcgc tttctcatag ctcacgctgt aggtatctca gttcggtgta ggtcgttcgc 420

tccaagctgg gctgtgtgca cgaacccccc gttcagcccg accgctgcgc cttatccggt 480

aactatcgtc ttgagtccaa cccggtaaga cacgacttat cgccactggc agcagccact 540

ggtaacagga ttagcagagc gaggtatgta ggcggtgcta cagagttctt gaagtggtgg 600

cctaactacg gctacactag aagaacagta tttggtatct gcgctctgct gaagccagtt 660

accttcggaa aaagagttgg tagctcttga tccggcaaac aaaccaccgc tggtagcggt 720

ggtttttttg tttgcaagca gcagattacg cgcagaaaaa aaggatctca agaagatcct 780

ttgatctttt ctacggggtc tgacgctcag tggaacgaaa actcacgtta agggattttg 840

gtcatgagat tatcaaaaag gatcttcacc tagatccttt taaattaaaa atgaagtttt 900

aaatcaatct aaagtatata tgagtaaact tggtctgaca gttaccaatg cttaatcagt 960

gaggcaccta tctcagcgat ctgtctattt cgttcatcca tagttgcctg actccccgtc 1020

gtgtagataa ctacgatacg ggagggctta ccatctggcc ccagtgctgc aatgataccg 1080

cgagacccac gctcaccggc tccagattta tcagcaataa accagccagc cggaagggcc 1140

gagcgcagaa gtggtcctgc aactttatcc gcctccatcc agtctattaa ttgttgccgg 1200

gaagctagag taagtagttc gccagttaat agtttgcgca acgttgttgc cattgctaca 1260

ggcatcgtgg tgtcacgctc gtcgtttggt atggcttcat tcagctccgg ttcccaacga 1320

tcaaggcgag ttacatgatc ccccatgttg tgcaaaaaag cggttagctc cttcggtcct 1380

ccgatcgttg tcagaagtaa gttggccgca gtgttatcac tcatggttat ggcagcactg 1440

cataattctc ttactgtcat gccatccgta agatgctttt ctgtgactgg tgagtactca 1500

accaagtcat tctgagaata gtgtatgcgg cgaccgagtt gctcttgccc ggcgtcaata 1560

cgggataata ccgcgccaca tagcagaact ttaaaagtgc tcatcattgg aaaacgttct 1620

tcggggcgaa aactctcaag gatcttaccg ctgttgagat ccagttcgat gtaacccact 1680

cgtgcaccca actgatcttc agcatctttt actttcacca gcgtttctgg gtgagcaaaa 1740

acaggaaggc aaaatgccgc aaaaaaggga ataagggcga cacggaaatg ttgaatactc 1800

atactcttcc tttttcaata ttattgaagc atttatcagg gttattgtct catgagcgga 1860

tacatatttg aatgtattta gaaaaataaa caaatagggg ttccgcgcac atttccccga 1920

aaagtgccac ctgacgtcta agaaaccatt attatcatga cattaaccta taaaaatagg 1980

cgtatcacga ggccctttcg tctcgcgcgt ttcggtgatg acggtgaaaa cctctgacac 2040

atgcagctcc cggagacggt cacagcttgt ctgtaagcgg atgccgggag cagacaagcc 2100

cgtcagggcg cgtcagcggg tgttggcggg tgtcggggct ggcttaacta tgcggcatca 2160

gagcagattg tactgagagt gcaccataaa attgtaaacg ttaatatttt gttaaaattc 2220

gcgttaaatt tttgttaaat cagctcattt tttaaccaat aggccgaaat cggcaaaatc 2280

ccttataaat caaaagaata gcccgagata gggttgagtg ttgttccagt ttggaacaag 2340

agtccactat taaagaacgt ggactccaac gtcaaagggc gaaaaaccgt ctatcagggc 2400

gatggcccac tacgtgaacc atcacccaaa tcaagttttt tggggtcgag gtgccgtaaa 2460

gcactaaatc ggaaccctaa agggagcccc cgatttagag cttgacgggg aaagccggcg 2520

aacgtggcga gaaaggaagg gaagaaagcg aaaggagcgg gcgctagggc gctggcaagt 2580

gtagcggtca cgctgcgcgt aaccaccaca cccgccgcgc ttaatgcgcc gctacagggc 2640

gcgtactatg gttgctttga cgtatgcggt gtgaaatacc gcacagatgc gtaaggagaa 2700

aataccgcat caggcgccat tcgccattca ggctgcgcaa ctgttgggaa gggcgatcgg 2760

tgcgggcctc ttcgctatta cgccagctgg cgaaaggggg atgtgctgca aggcgattaa 2820

gttgggtaac gccagggttt tcccagtcac gacgttgtaa aacgacggcc agtgaattcg 2880

aatggccatg gaggatttat tctctccgca gttttgagct taatcgtctc attaactact 2940

gcacttttct ccgattggct tcgtcggaga aaataatcta acggcatgaa gcgcaaatta 3000

aaagccactc aactatgggc ctagttgggt ggcttttttg caaccattac ggttttactt 3060

ctctataaga attatagcat caatcttcat cattagccaa ctcaatagaa gctcgatata 3120

gatcatgttg agctttatcc aagctatcta aaagttcgcc ataaatatca atatttttct 3180

tttcagcaac caattttttc aaatcttgaa gattatcagc aatctgttct gccataactc 3240

catagatttc agcttgtttc ttcatattgt caattgtctt aatatcttgt aactccattc 3300

ctatcactcc catcttacat agttacttac cttagaattc caacgatata tcacagtatc 3360

tacaacatca tctttttctt tttcgtcatc cacattaatt agatcatctt ctttttcgtc 3420

cagcaataac tcatgatgaa tctcttttag taatcccgca aagcttaatt gtcgagttcc 3480

agccagagca tgctcgagct cgtcaacaac aactaaatcg cctttctcat catccgtaat 3540

ataatcataa tctttaactt gatatttact tacttctttt gccgaagcaa tcaaactatt 3600

atcttttttt gacgcccgaa ttttcttgat attaacaatc ggcttgtagt ctaatttcct 3660

tgctcgcttc cataattgtg accaattctc ttgggtaaga taatgaccct tagaaaaata 3720

actagatttc accattaaca aaatatgtac atgttgatgg aaagttagac tatctcgatt 3780

gaccgttatt tctgtagatc gcacataacc aagaagatct ttttttacat ctttatattg 3840

aaaaagccga taaattgaac gattcatcga agtaagattt tttctaagct cgcccaactt 3900

tgagctttcc tcagttaatg tcaagaacag aaatatagca ctaggatcct ttttatatgc 3960

tgtatctaaa atttgacgca actcataaga atttttcatc gatcggcgcc agctacataa 4020

tggacaaagt ctcgaatgac aaaaccacgt ttgatataac tttaaaccat tatcagtttt 4080

cgcaaattga agaacttctc cgcactttga aacgttatga gcttttttaa aatttaaaac 4140

ctccagatat tgggaataag ttaagttagc aagttttttc tgcttccacg gtctaacttt 4200

cccattacta gaggtatctt ttagaatttc ttttttatac atttcttata cctctaactg 4260

caaaaactac aagcagtcag agttagttat cattgatata ttcctaatga catattataa 4320

tagggttcga agttaataac gataactaac tcatgcatcg acggccaaat cgaacaactg 4380

catgagtttt ttatttaatt cttacaatgc ttattataca ccaaaaagtg ttatatttta 4440

aaatcctaat aaacccagtc atatcaaggg tttaacccaa tttcaaaaaa tggcgttgtt 4500

tctatatgta tcaagataag aagaaactcg cttacagcga gtttctaaat aggggtttca 4560

cccctaaaac cctgaccagc tgagccagct ggaccgcatt tcccctcgcc gggcgggcgt 4620

aaagcggaaa aaatgacttt tttctcgctt tcgctcaact agggtcttcc cgtctcacca 4680

tgtcaagcgc tgcccttccg ctacgctcca ggcgtttgtc gcttgacact cacctactaa 4740

cagcgggctt cgccaccagt ctactatctg caacacggca acaagattaa tcagaatatg 4800

cattgaagca gtgcgctccg ctccttgtct tgcgacaaag cgagcgcact gcttcaatgc 4860

ttttttattt tctgaattcc ataactcgat aattagaaaa agttagtccg ttagaaacgt 4920

ctgccacgcc gaggaagtca ttccagtttg accactcaaa tcatcagcga catcattgag 4980

ataatcaatc agcaattttc tagtttctat ctgctttgga atttttacca cagccaaaat 5040

tgagtcccac ttagcaatca caaaagattt tctgacggct ttattagcct tttccgcact 5100

aatatttttc gctttatctc gtttattttg atcggtcgat ggtatccgat cgactgttgt 5160

tacagcattt gacgcaggtt gctagataaa cgactagacc gaataatttt tattccaact 5220

atttttgcaa ccaaaaaaat cacaaaataa tcaaccaata aaacgataac taacttcttg 5280

ctacatccga ttaatttttc tccagcctgc cagaaatcgg cccaaatatt gaaatattga 5340

ggaaattttg ggctgaaaaa aacattaatt ttttcggcaa acaagccaac acagccaagc 5400

agaaaagcta agaaaataag ttccagccaa ttaaaaaatc gccacgaaag agaaaaaaat 5460

cggtcaaaat aaatcgaact tgatttcatt ttctagcccc cgaagtcttc gataaacggc 5520

gccataaaag gaactacacg gttttccggt tgagtacttg caagccctaa ccccttagaa 5580

aagccgtcat gattaacaac tgttgtcgac ttttttttga taagttaagg gatgcagttt 5640

atgcacccct taacttatca ttattagtgt aaacaaggaa ggatatcttg tacggcttga 5700

acaattacac caccagcagc gatatcagca gaaatacgag caatatgttc gtcaagtcct 5760

tgttcgttat cgttccaaat atatggccga tctgatgtag gtttttggcc gacaacatga 5820

cgtactgctg aataaagacg ttctgtagct ggacttaaac aagcacgagc atcataatgg 5880

cccgtctttt tgtaagtccg taaatcaaca gcttgtactc caaacattaa tgcaatagct 5940

acgtagtttt gaaaaatatc aacagaccgt cgtgctaacg tagcagaagt gtatccttga 6000

gagttaatat tttggttaaa ttgttcggcg tgtgtaggaa agcgatcagc aattgaattt 6060

ccgtaaaaag tcaacaatgg cataattgag ttgccacaaa tttgcaaacc cttaagaccc 6120

atgtttacct ttcgttcccg gtttcccaat aatgatggtg gaagtccgtt actgaattct 6180

ggactggcca aaagtgcaat ttggacatcc aaatgctttg caagaagacc aatgtagtaa 6240

cgtaagtggt ccatgcccat gccaacatat tgacccaaaa agttaccgcc atggtatgag 6300

gcttggttat caacgtcaat taatggattg tcagtgacac tattaatttc gatttcaatt 6360

tgcttagcaa tttgtgaaat tccatcaaca ataggaccca aatattgtgg taagcaacgt 6420

aaactatagc ggtcttgaat aagttcatga tcacgataat catgcttacc atctaattcg 6480

tcgcgaacaa gttgactatt agctaaaaga gaaatcattt gatcagcagc ccaaagttga 6540

cctgggtgag gtttagaatt gtgaatgaat gggtggaaac tttgatttgt gccattaagt 6600

gcttgaatat ctaaggcatg aacacccatg gcaatagcag taagaatttg agtatcataa 6660

acgcaattag cagcaattcc cgtcataact gatgtaccat tcatcatagc aagaccttct 6720

tttggaagta atgtaagtgg tgataaattt aattgtcgaa gagctgttgg ggcatccatt 6780

tctttaccat taaaatcaac tttgaatgat ggatccaaac caattaaaga acctgtaata 6840

taagataaag gaacaagatc tccacttgca ccaattgatc cgaattcgta aacataaggt 6900

gtaacaccgg cgttaaggaa aatttccata cgcttaatta attcaagacg aataccagaa 6960

gcgccacgca tgtgtgaatt tgcacgtaat aacatagcag cacgaacatc tgctaatgga 7020

agtttattac cagctcctgt tttaagaaac caaacaagat tagtttgaag ttcagaagct 7080

tgttcacgac taatagcgac gtttgccatt ccaccgaatc ctgaagtaac accataaatt 7140

ggttctcctg attcaacagc gttattgata tagtcgcaac tagcttgaat gccttgtaaa 7200

atgtcagtgt tgttagttaa actaactaaa gtaccattgc gtgcaacccg tgcaacatcg 7260

ttaatagtaa gcttttgatt accaatgata acattagcac ttgagtttcc agtaaatgag 7320

aattgttgag aactagtctt agattgtgct tgagataaag tcttcattta tacctactcc 7380

ttcttaatta caaattttta gcatctaatt taacttcaat tcctattata caaaatttta 7440

agatactgca ctatcaacac actcttgaac atatgcatcc taggtcgtct attaatactt 7500

ccaggagtat acgtagccag gctaacgtta atcaaccagg tatcctctag aactatagct 7560

agcatgccta tatatggatc caattgcaat gatcatcatg acagatctgc gcgcgatcga 7620

tatcagcgct ttaaatttgc gcatgctagc tatagttcta gaggtaccgg ttgttaacgt 7680

tagccggcta cgtatactcc ggaatattaa taggcctagg atgcatatgc ttagaagcaa 7740

acttaagagt gtgttgatag tgcagtatct taaaattttg tataatagga attgaagtta 7800

aattagatgc taaaaatttg taattaagaa ggagtgatta catgaacaaa aatataaaat 7860

attctcaaaa ctttttaacg agtgaaaaag tactcaacca aataataaaa caattgaatt 7920

taaaagaaac cgataccgtt tacgaaattg gaacaggtaa agggcattta acgacgaaac 7980

tggctaaaat aagtaaacag gtaacgtcta ttgaattaga cagtcatcta ttcaacttat 8040

cgtcagaaaa attaaaactg aatactcgtg tcactttaat tcaccaagat attctacagt 8100

ttcaattccc taacaaacag aggtataaaa ttgttgggag tattccttac catttaagca 8160

cacaaattat taaaaaagtg gtttttgaaa gccatgcgtc tgacatctat ctgattgttg 8220

aagaaggatt ctacaagcgt accttggata ttcaccgaac actagggttg ctcttgcaca 8280

ctcaagtctc gattcagcaa ttgcttaagc tgccagcgga atgctttcat cctaaaccaa 8340

aagtaaacag tgtcttaata aaacttaccc gccataccac agatgttcca gataaatatt 8400

ggaagctata tacgtacttt gtttcaaaat gggtcaatcg agaatatcgt caactgttta 8460

ctaaaaatca gtttcatcaa gcaatgaaac acgccaaagt aaacaattta agtaccgtta 8520

cttatgagca agtattgtct atttttaata gttatctatt atttaacggg aggaaataat 8580

tctatgagtc gcttttgtaa atttggaaag ttacacgtta ctaaagggaa tgtagataaa 8640

ttattaggta tactactgac agcttccaag catatggcgg ccgcctgcag ctggcgccat 8700

cgatacgcgt acgtcgcgac cgcggacatg tacagagctc gagaagtact agtggccacg 8760

tgggccgtgc accttaagct tggcgtaatc atggtcatag ctgtttcctg tgtgaaattg 8820

ttatccgctc acaattccac acaacatacg agccggaagc ataaagtgta aagcctgggg 8880

tgcctaatga gtgagctaac tcacattaat tgcgttgcgc tcactgcccg ctttccagtc 8940

gggaaacctg tcgtgccagc tgcattaatg aatcggccaa cgcgcgggga gaggcggttt 9000

gcgtattggg cgc 9013

<210> 14
<211> 9142
<212> DNA
<213> Artificial Sequence

<220>
<223> pHENOMM-sec

<400> 14
tcttccgctt cctcgctcac tgactcgctg cgctcggtcg ttcggctgcg gcgagcggta 60

tcagctcact caaaggcggt aatacggtta tccacagaat caggggataa cgcaggaaag 120

aacatgtgag caaaaggcca gcaaaaggcc aggaaccgta aaaaggccgc gttgctggcg 180

tttttccata ggctccgccc ccctgacgag catcacaaaa atcgacgctc aagtcagagg 240

tggcgaaacc cgacaggact ataaagatac caggcgtttc cccctggaag ctccctcgtg 300

cgctctcctg ttccgaccct gccgcttacc ggatacctgt ccgcctttct cccttcggga 360

agcgtggcgc tttctcatag ctcacgctgt aggtatctca gttcggtgta ggtcgttcgc 420

tccaagctgg gctgtgtgca cgaacccccc gttcagcccg accgctgcgc cttatccggt 480
aactatcgtc ttgagtccaa cccggtaaga cacgacttat cgccactggc agcagccact 540
ggtaacagga ttagcagagc gaggtatgta ggcggtgcta cagagttctt gaagtggtgg 600
cctaactacg gctacactag aagaacagta tttggtatct gcgctctgct gaagccagtt 660
accttcggaa aaagagttgg tagctcttga tccggcaaac aaaccaccgc tggtagcggt 720
ggtttttttg tttgcaagca gcagattacg cgcagaaaaa aaggatctca agaagatcct 780
ttgatctttt ctacggggtc tgacgctcag tggaacgaaa actcacgtta agggattttg 840
gtcatgagat tatcaaaaag gatcttcacc tagatccttt taaattaaaa atgaagtttt 900
aaatcaatct aaagtatata tgagtaaact tggtctgaca gttaccaatg cttaatcagt 960
gaggcaccta tctcagcgat ctgtctattt cgttcatcca tagttgcctg actccccgtc 1020
gtgtagataa ctacgatacg ggagggctta ccatctggcc ccagtgctgc aatgataccg 1080
cgagacccac gctcaccggc tccagattta tcagcaataa accagccagc cggaagggcc 1140
gagcgcagaa gtggtcctgc aactttatcc gcctccatcc agtctattaa ttgttgccgg 1200
gaagctagag taagtagttc gccagttaat agtttgcgca acgttgttgc cattgctaca 1260
ggcatcgtgg tgtcacgctc gtcgtttggt atggcttcat tcagctccgg ttcccaacga 1320
tcaaggcgag ttacatgatc ccccatgttg tgcaaaaaag cggttagctc cttcggtcct 1380
ccgatcgttg tcagaagtaa gttggccgca gtgttatcac tcatggttat ggcagcactg 1440
cataattctc ttactgtcat gccatccgta agatgctttt ctgtgactgg tgagtactca 1500
accaagtcat tctgagaata gtgtatgcgg cgaccgagtt gctcttgccc ggcgtcaata 1560
cgggataata ccgcgccaca tagcagaact ttaaaagtgc tcatcattgg aaaacgttct 1620
tcggggcgaa aactctcaag gatcttaccg ctgttgagat ccagttcgat gtaacccact 1680
cgtgcaccca actgatcttc agcatctttt actttcacca gcgtttctgg gtgagcaaaa 1740
acaggaaggc aaaatgccgc aaaaaaggga ataagggcga cacggaaatg ttgaatactc 1800
atactcttcc tttttcaata ttattgaagc atttatcagg gttattgtct catgagcgga 1860
tacatatttg aatgtattta gaaaaataaa caaatagggg ttccgcgcac atttccccga 1920
aaagtgccac ctgacgtcta agaaaccatt attatcatga cattaaccta taaaaatagg 1980
cgtatcacga ggccctttcg tctcgcgcgt ttcggtgatg acggtgaaaa cctctgacac 2040
atgcagctcc cggagacggt cacagcttgt ctgtaagcgg atgccgggag cagacaagcc 2100
cgtcagggcg cgtcagcggg tgttggcggg tgtcggggct ggcttaacta tgcggcatca 2160
gagcagattg tactgagagt gcaccataaa attgtaaacg ttaatatttt gttaaaattc 2220
gcgttaaatt tttgttaaat cagctcattt tttaaccaat aggccgaaat cggcaaaatc 2280

ccttataaat caaaagaata gcccgagata gggttgagtg ttgttccagt ttggaacaag 2340

agtccactat taaagaacgt ggactccaac gtcaaagggc gaaaaaccgt ctatcagggc 2400

gatggcccac tacgtgaacc atcacccaaa tcaagttttt tggggtcgag gtgccgtaaa 2460

gcactaaatc ggaaccctaa agggagcccc cgatttagag cttgacgggg aaagccggcg 2520

aacgtggcga gaaaggaagg gaagaaagcg aaaggagcgg gcgctagggc gctggcaagt 2580

gtagcggtca cgctgcgcgt aaccaccaca cccgccgcgc ttaatgcgcc gctacagggc 2640

gcgtactatg gttgctttga cgtatgcggt gtgaaatacc gcacagatgc gtaaggagaa 2700

aataccgcat caggcgccat tcgccattca ggctgcgcaa ctgttgggaa gggcgatcgg 2760

tgcgggcctc ttcgctatta cgccagctgg cgaaaggggg atgtgctgca aggcgattaa 2820

gttgggtaac gccagggttt tcccagtcac gacgttgtaa aacgacggcc agtgaattcg 2880

aatggccatg gaggatttat tctctccgca gttttgagct taatcgtctc attaactact 2940

gcacttttct ccgattggct tcgtcggaga aaataatcta acggcatgaa gcgcaaatta 3000

aaagccactc aactatgggc ctagttgggt ggcttttttg caaccattac ggttttactt 3060

ctctataaga attatagcat caatcttcat cattagccaa ctcaatagaa gctcgatata 3120

gatcatgttg agctttatcc aagctatcta aaagttcgcc ataaatatca atatttttct 3180

tttcagcaac caattttttc aaatcttgaa gattatcagc aatctgttct gccataactc 3240

catagatttc agcttgtttc ttcatattgt caattgtctt aatatcttgt aactccattc 3300

ctatcactcc catcttacat agttacttac cttagaattc caacgatata tcacagtatc 3360

tacaacatca tctttttctt tttcgtcatc cacattaatt agatcatctt ctttttcgtc 3420

cagcaataac tcatgatgaa tctcttttag taatcccgca aagcttaatt gtcgagttcc 3480

agccagagca tgctcgagct cgtcaacaac aactaaatcg cctttctcat catccgtaat 3540

ataatcataa tctttaactt gatatttact tacttctttt gccgaagcaa tcaaactatt 3600

atcttttttt gacgcccgaa ttttcttgat attaacaatc ggcttgtagt ctaatttcct 3660

tgctcgcttc cataattgtg accaattctc ttgggtaaga taatgaccct tagaaaaata 3720

actagatttc accattaaca aaatatgtac atgttgatgg aaagttagac tatctcgatt 3780

gaccgttatt tctgtagatc gcacataacc aagaagatct ttttttacat ctttatattg 3840

aaaaagccga taaattgaac gattcatcga agtaagattt tttctaagct cgcccaactt 3900

tgagctttcc tcagttaatg tcaagaacag aaatatagca ctaggatcct ttttatatgc 3960

tgtatctaaa atttgacgca actcataaga atttttcatc gatcggcgcc agctacataa 4020

tggacaaagt ctcgaatgac aaaaccacgt ttgatataac tttaaaccat tatcagtttt 4080

cgcaaattga agaacttctc cgcactttga aacgttatga gcttttttaa aatttaaaac 4140

ctccagatat tgggaataag ttaagttagc aagttttttc tgcttccacg gtctaacttt 4200

cccattacta gaggtatctt ttagaatttc ttttttatac atttcttata cctctaactg 4260

caaaaactac aagcagtcag agttagttat cattgatata ttcctaatga catattataa 4320

tagggttcga agttaataac gataactaac tcatgcatcg acggccaaat cgaacaactg 4380

catgagtttt ttatttaatt cttacaatgc ttattataca ccaaaaagtg ttatatttta 4440

aaatcctaat aaacccagtc atatcaaggg tttaacccaa tttcaaaaaa tggcgttgtt 4500

tctatatgta tcaagataag aagaaactcg cttacagcga gtttctaaat aggggtttca 4560

cccctaaaac cctgaccagc tgagccagct ggaccgcatt tcccctcgcc gggcgggcgt 4620

aaagcggaaa aaatgacttt tttctcgctt tcgctcaact agggtcttcc cgtctcacca 4680

tgtcaagcgc tgcccttccg ctacgctcca ggcgtttgtc gcttgacact cacctactaa 4740

cagcgggctt cgccaccagt ctactatctg caacacggca acaagattaa tcagaatatg 4800

cattgaagca gtgcgctccg ctccttgtct tgcgacaaag cgagcgcact gcttcaatgc 4860

tttttatttt tctgaattcc ataactcgat aattagaaaa agttagtccg ttagaaacgt 4920

ctgccacgcc gaggaagtca ttccagtttg accactcaaa tcatcagcga catcattgag 4980

ataatcaatc agcaattttc tagtttctat ctgctttgga atttttacca cagccaaaat 5040

tgagtcccac ttagcaatca caaaagattt tctgacggct ttattagcct tttccgcact 5100

aatatttttc gctttatctc gtttattttg atcggtcgat ggtatccgat cgactgttgt 5160

tacagcattt gacgcaggtt gctagataaa cgactagacc gaataatttt tattccaact 5220

atttttgcaa ccaaaaaaat cacaaaataa tcaaccaata aaacgataac taacttcttg 5280

ctacatccga ttaatttttc tccagcctgc cagaaatcgg cccaaatatt gaaatattga 5340

ggaaattttg ggctgaaaaa aacattaatt ttttcggcaa acaagccaac acagccaagc 5400

agaaaagcta agaaaataag ttccagccaa ttaaaaaatc gccacgaaag agaaaaaaat 5460

cggtcaaaat aaatcgaact tgatttcatt ttctagcccc cgaagtcttc gataaacggc 5520

gccataaaag gaactacacg gttttccggt tgagtacttg caagccctaa ccccttagaa 5580

aagccgtcat gattaacaac tgttgtcgac ttttttttga taagttaagg gatgcagttt 5640

atgcacccct taacttatca ttattagtgt aaacaaggaa ggatatcttg tacggcttga 5700

acaattacac caccagcagc gatatcagca gaaatacgag caatatgttc gtcaagtcct 5760

tgttcgttat cgttccaaat atatggccga tctgatgtag gtttttggcc gacaacatga 5820

cgtactgctg aataaagacg ttctgtagct ggacttaaac aagcacgagc atcataatgg 5880

cccgtctttt tgtaagtccg taaatcaaca gcttgtactc caaacattaa tgcaatagct 5940

acgtagtttt gaaaaatatc aacagaccgt cgtgctaacg tagcagaagt gtatccttga 6000

gagttaatat tttggttaaa ttgttcggcg tgtgtaggaa agcgatcagc aattgaattt 6060

ccgtaaaaag tcaacaatgg cataattgag ttgccacaaa tttgcaaacc cttaagaccc 6120

atgtttacct ttcgttcccg gtttcccaat aatgatggtg gaagtccgtt actgaattct 6180

ggactggcca aaagtgcaat ttggacatcc aaatgctttg caagaagacc aatgtagtaa 6240

cgtaagtggt ccatgcccat gccaacatat tgacccaaaa agttaccgcc atggtatgag 6300

gcttggttat caacgtcaat taatggattg tcagtgacac tattaatttc gatttcaatt 6360

tgcttagcaa tttgtgaaat tccatcaaca ataggaccca aatattgtgg taagcaacgt 6420

aaactatagc ggtcttgaat aagttcatga tcacgataat catgcttacc atctaattcg 6480

tcgcgaacaa gttgactatt agctaaaaga gaaatcattt gatcagcagc ccaaagttga 6540

cctgggtgag gtttagaatt gtgaatgaat gggtggaaac tttgatttgt gccattaagt 6600

gcttgaatat ctaaggcatg aacacccatg gcaatagcag taagaatttg agtatcataa 6660

acgcaattag cagcaattcc cgtcataact gatgtaccat tcatcatagc aagaccttct 6720

tttggaagta atgtaagtgg tgataaattt aattgtcgaa gagctgttgg ggcatccatt 6780

tctttaccat taaaatcaac tttgaatgat ggatccaaac caattaaaga acctgtaata 6840

taagataaag gaacaagatc tccacttgca ccaattgatc cgaattcgta aacataaggt 6900

gtaacaccgg cgttaaggaa aatttccata cgcttaatta attcaagacg aataccagaa 6960

gcgccacgca tgtgtgaatt tgcacgtaat aacatagcag cacgaacatc tgctaatgga 7020

agtttattac cagctcctgt tttaagaaac caaacaagat tagtttgaag ttcagaagct 7080

tgttcacgac taatagcgac gtttgccatt ccaccgaatc ctgaagtaac accataaatt 7140

ggttctcctg attcaacagc gttattgata tagtcgcaac tagcttgaat gccttgtaaa 7200

atgtcagtgt tgttagttaa actaactaaa gtaccattgc gtgcaacccg tgcaacatcg 7260

ttaatagtaa gcttttgatt accaatgata acattagcac ttgagtttcc agtaaatgag 7320

aattgttgag aactagtctt agattgtgct tgagataaag tcttcattta tacctactcc 7380

tatcaactga agaagcatca acaaccataa tattttggct tacgccccaa aaactaataa 7440

cgaccaataa tggtaatagc cacacaggaa gtccgttacg gtgatgttta cctgtatttt 7500

cagtatacat tcttaattac aaatttttag catctaattt aacttcaatt cctattatac 7560

aaaattttaa gatactgcac tatcaacaca ctcttgaaca tatgcatcct aggtcgtcta 7620

ttaatacttc caggagtata cgtagccagg ctaacgttaa tcaaccaggt atcctctaga 7680

actatagcta gcatgcctat atatggatcc aattgcaatg atcatcatga cagatctgcg 7740

cgcgatcgat atcagcgctt taaatttgcg catgctagct atagttctag aggtaccggt 7800

tgttaacgtt agccggctac gtatactccg gaatattaat aggcctagga tgcatatgct 7860

tagaagcaaa cttaagagtg tgttgatagt gcagtatctt aaaattttgt ataataggaa 7920

ttgaagttaa attagatgct aaaaatttgt aattaagaag gagtgattac atgaacaaaa 7980

atataaaata ttctcaaaac tttttaacga gtgaaaaagt actcaaccaa ataataaaac 8040

aattgaattt aaaagaaacc gataccgttt acgaaattgg aacaggtaaa gggcatttaa 8100

cgacgaaact ggctaaaata agtaaacagg taacgtctat tgaattagac agtcatctat 8160

tcaacttatc gtcagaaaaa ttaaaactga atactcgtgt cactttaatt caccaagata 8220

ttctacagtt tcaattccct aacaaacaga ggtataaaat tgttgggagt attccttacc 8280

atttaagcac acaaattatt aaaaaagtgg tttttgaaag ccatgcgtct gacatctatc 8340

tgattgttga agaaggattc tacaagcgta ccttggatat tcaccgaaca ctagggttgc 8400

tcttgcacac tcaagtctcg attcagcaat tgcttaagct gccagcggaa tgctttcatc 8460

ctaaaccaaa agtaaacagt gtcttaataa aacttacccg ccataccaca gatgttccag 8520

ataaatattg gaagctatat acgtactttg tttcaaaatg ggtcaatcga gaatatcgtc 8580

aactgtttac taaaaatcag tttcatcaag caatgaaaca cgccaaagta aacaatttaa 8640

gtaccgttac ttatgagcaa gtattgtcta tttttaatag ttatctatta tttaacggga 8700

ggaaataatt ctatgagtcg cttttgtaaa tttggaaagt tacacgttac taaagggaat 8760

gtagataaat tattaggtat actactgaca gcttccaagc atatggcggc cgcctgcagc 8820

tggcgccatc gatacgcgta cgtcgcgacc gcggacatgt acagagctcg agaagtacta 8880

gtggccacgt gggccgtgca ccttaagctt ggcgtaatca tggtcatagc tgtttcctgt 8940

gtgaaattgt tatccgctca caattccaca caacatacga gccggaagca taaagtgtaa 9000

agcctggggt gcctaatgag tgagctaact cacattaatt gcgttgcgct cactgcccgc 9060

tttccagtcg ggaaacctgt cgtgccagct gcattaatga atcggccaac gcgcggggag 9120

aggcggtttg cgtattgggc gc 9142

<210> 15
<211> 1963
<212> DNA
<213> Artificial Sequence

<220>
<223> synthesized

<400> 15
gcatgctagc tatagttcta gaggatacct ggttgattaa cgttagcctg gctacgtata 60

ctcctggaag tattaataga cgacctagga tgcatatgtt caagagtgtg ttgatagtgc 120

agtatcttaa aattttgtat aataggaatt gaagttaaat tagatgctaa aaatttgtaa 180

ttaagaagga gtaggtataa atgaagactt tatctcaagc acaatctaag actagttctc 240

aacaattctc atttactgga aactcaagtg ctaatgttat cattggtaat caaaagctta 300

ctattaacga tgttgcacgg gttgcacgca atggtacttt agttagttta actaacaaca 360

ctgacatttt acaaggcatt caagctagtt gcgactatat caataacgct gttgaatcag 420

gagaaccaat ttatggtgtt acttcaggat tcggtggaat ggcaaacgtc gctattagtc 480

gtgaacaagc ttctgaactt caaactaatc ttgtttggtt tcttaaaaca ggagctggta 540

ataaacttcc attagcagat gttcgtgctg ctatgttatt acgtgcaaat tcacacatgc 600

gtggcgcttc tggtattcgt cttgaattaa ttaagcgtat ggaaattttc cttaacgccg 660

gtgttacacc ttatgtttac gaattcggat caattggtgc aagtggagat cttgttcctt 720

tatcttatat tacaggttct ttaattggtt tggatccatc attcaaagtt gattttaatg 780

gtaaagaaat ggatgcccca acagctcttc gacaattaaa tttatcacca cttacattac 840

ttccaaaaga aggtcttgct atgatgaatg gtacatcagt tatgacggga attgctgcta 900

attgcgttta tgatactcaa attcttactg ctattgccat gggtgttcat gccttagata 960

ttcaagcact taatggcaca aatcaaagtt tccacccatt cattcacaat tctaaacctc 1020

acccaggtca actttgggct gctgatcaaa tgatttctct tttagctaat agtcaacttg 1080

ttcgcgacga attagatggt aagcatgatt atcgtgatca tgaacttatt caagaccgct 1140

atagtttacg ttgcttacca caatatttgg gtcctattgt tgatggaatt tcacaaattg 1200

ctaagcaaat tgaaatcgaa attaatagtg tcactgacaa tccattaatt gacgttgata 1260

accaagcctc ataccatggc ggtaactttt tgggtcaata tgttggcatg ggcatggacc 1320

acttacgtta ctacattggt cttcttgcaa agcatttgga tgtccaaatt gcacttttgg 1380

ccagtccaga attcagtaac ggacttccac catcattatt gggaaaccgg gaacgaaagg 1440

taaacatggg tcttaagggt ttgcaaattt gtggcaactc aattatgcca ttgttgactt 1500

tttacggaaa ttcaattgct gatcgctttc ctacacacgc cgaacaattt aaccaaaata 1560

ttaactctca aggatacact tctgctacgt tagcacgacg gtctgttgat atttttcaaa 1620

actacgtagc tattgcatta atgtttggag tacaagctgt tgatttacgg acttacaaaa 1680

agacgggcca ttatgatgct cgtgcttgtt taagtccagc tacagaacgt ctttattcag 1740

cagtacgtca tgttgtcggc caaaaaccta catcagatcg gccatatatt tggaacgata 1800

acgaacaagg acttgacgaa catattgctc gtatttctgc tgatatcgct gctggtggtg 1860

taattgttca agccgtacaa gatatccttc cttgtttaca ctaataatga taagttaagg 1920

ggtgcataaa ctgcatccct taacttatca aaaaaaagtc gac 1963

## Description

### BACKGROUND

The present invention relates to a genetically modified probiotic (GMP), and more specifically to a GMP adapted to provide the phenylalanine ammonia-lyase (PAL) gene or other phenylalanine-degrading enzyme for the treatment of phenylketonuria (PKU). The GMP is herein referred to as PHEnominal.

### Advantages Over Conventional Technologies

PKU is a disease that renders the patient incapable of digesting the amino acid phenylalanine, resulting in a subsequent accumulation in the blood causing toxicity.

Several technologies have been developed for the treatment of PKU. First, liver and/or hepatocyte transplants have been used. A regular transplant is not considered an acceptable treatment by insurance companies as the cost of treatment for PKU transfers to the cost of treatment to prevent organ rejection.

Second, gene therepy techniques have been utilized for the treatment of PKU. Gene therapy methods have yet to break the barrier of gene silencing. In gene silencing, a eukaryotic cell detects genes of a viral source, such as a gene therapy vector, and methylates the DNA to silence it. Additionally, after the "Bubble boy" syndrome incident (children developing leukemia following gene therapy for a terminal illness), gene therapy has not gained wide spread acceptance or regulatory approval in the USA.

Finally, several other treatments have been tried, without the level of cost-effectiveness required for wide-spread adoption.

Other treatments for PKU patients are made difficult by the many co-factors and co-enzymes required for PAH (the deficient enzyme) to function. Current dietary therapies cost approximately $40,000.00 per year for a teen or adult. Co-factor therapy, which supplies an excess of PAH's co-factor, reduces phenylalanine (phe) in some of the patients. However many patients (40 - 50%) see no improvements, or improvement is not sufficient to warrant the cost of co-factor therapy. The cofactor therapy by the name of Kuvan®, is also very expensive ($40,000.00 per year for an adult or teen). Responses from this treatment range from no change to complete control of phenylalanine levels. It is thought that the patient's level of response to this therapy is dictated by the exact genetic mutation they carry.

PEG-PAL, is currently under clinical trials. For this treatment, PAL is produced by bacteria, purified, and coated in polyethylene glycol (PEG). The resulting PEG-PAL is injected subcutaneously into the patient, and enters the blood stream. Once in the blood, the PEG coating helps protect PAL from immunodetection while allowing the enzyme to degrade serum phenylalanine. Although cost estimates have not been released, similar therapies (PEG-Adenosine deaminase) are quite expensive. Additionally, complications of this therapy have been detected in phase 2 clinical trials.

Yeast PAL in microcapsules has also been studied as a potential treatment. This study used rats injected with phenylalanine rather than a true disease model. Yeast PAL has lower activity (cannot compare exactly, due to notation used), and of further concern, yeast PAL is nearly as good at catabolizing tyrosine as phe (.67 micromoles/min per unit enzyme for tyrosine and 1.4 micromoles/min per unit enzyme at catabolizing phe). The latter is of particular concern since PKU patients also are unable to synthesize the essential amino acid tyrosine from phe. Additionally, microcapsules were deemed too expensive to use as a PKU therapy.

Oral naked PAL enzyme has been developed, but was only used in mice due to the restrictions of treatment. Namely, the PAL enzyme could not survive the stomach to arrive in the intestine unless it was given by gastric gavage along with enough sodium bicarbonate to neutralize the stomach acid. Furthermore, once in the intestine, PAL is cleaved rapidly by proteases such as trypsin and chymotrypsin.

The only current option known to work as a treatment in all patients with PKU is dietary restriction of the ingestion of foods containing phe. No natural protein is free of phe, and only a handful of proteins have been identified as having low phe contents. To this end, restricting phe ingestion can be fostered by the ingestion of synthetic foods. These foods are reported as having repugnant odor and flavor, resulting in poor dietary compliance. In addition to a diet more limited than that of a person with gluten intolerance, and the cost of the synthetic food diet is similar to that of the co-factor therapy ($1,000.00 per month for infant formula, with costs increasing as the patient becomes an adult and requires more food). Insurance companies commonly deny assistance with the food expenses of this treatment.

The invention described herein meets the need for a cost-effective therapy for PKU, with tolerable side-effects and high efficacy.

### SUMMARY

The present invention relates to a GMP adapted to provide the PAL gene or other phenylalanine-degrading enzyme for the treatment of PKU. In certain embodiments, the GMP of the present invention comprises a probiotic, a PAL gene to be expressed using the probiotic, wherein the PAL gene is functionally attached to a promoter and a ribosome binding site, and may be codon-optimized for expression in a certain host organism.

The present invention further comprises a method of treating the metabolic disease of PKU by oral administration and ingestion of a GMP. The GMP described herein will produce the enzyme PAL in the intestine when administered orally. The genetically engineered PAL will digest ingested phenylalanine contained in most proteinous foods. The end result will be that phenylalanine obtained from ingestion is not absorbed into the blood stream and cannot build up to toxic levels.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
FIGURE 1 shows the creation of the final functional mouse therapy shuttle vector starting with A) the *E. coli* plasmid pSLER1, B) addition of the pGT232 fragment to create shuttle vector ability, and C) final addition of FuzErmAvPAL for AvPAL expression when in a probiotic host; and
FIGURE 2 shows trans-cinnamic acid production by plasmid carried/100-23 cell line in an embodiment of the present invention.

### DETAILED DESCRIPTION OF PREFERRED EMBODIMENTS

One aspect of the present invention pertains to a genetically modified probiotic (GMP) to deliver functional PAL enzyme or other phenylalanine-degrading enzyme to the body of an animal, preferably to the small intestine. In certain embodiments, the probiotic is/has 1) capable of surviving the acid of the stomach and maintaining functionality after exiting the stomach; 2) capable of maintaining functionality and metabolic activity in the small intestine, and expressing the PAL gene or other phenylalanine-degrading enzyme in the intestine; 3) a mouse trophic and human trophic strain; 4) safe for oral use as a human probiotic; 5) capable of being produced in-vitro; and 6) amendable to electropration or another form of transformation.

In some embodiments, the probiotic capable of delivering functional phenylalanine-degrading enzyme, such as PAL enzyme, is useful in the treatment of PKU. If phenylalanine is pulled from the intestinal lumen by probiotics to be degraded by bacterial cells, phe will not enter the blood and will be unable to cause its pathogenic effects in a PKU animal/patient.

In certain embodiments, the probiotic is any of the genera of probiotic bacteria. Examples of probiotics include, but are not limited to, the *Lactobacilli* and *Lactococci* genera, which are used in yougurts and probiotic supplements in the USA, and would serve well as a safe delivery system to treat disease. Strain specificity may be selected based on the organism in which the treatment is occurring (i.e. a mouse specific strain may be selected for mouse *in-vivo* experimentation, while a human specific strain may be selected for human clinical trials and human treatment).

Because amino acid absorption occurs in the small intestine, a probiotic may be selected that is metabolically active in, and preferentially lives in the small intestine. Strains of *Lactobacillus reuteri* exist for mouse and human, and the microbe adheres to mucus of small intestine in both cases. Finally, a high *in vivo* retention rate of foreign DNA in this organism can be achieved by using a specific plasmid section in the final shuttle vector. Integration of the desired gene into the chromosome may also be performed if desired.

The enzyme for phenylalanine (phe) degradation may be selected from the enzyme family of Phenylalanine Ammonia Lysases (PALs). PAL enzymes cleave phenylalanine into trans-cinnamic acid and ammonia, both of which are safely and readily cleared from the mammalian body. Unlike other phe catabolizing enzymes, PAL enzymes do not require co-factors or co-enzymes to fuction. Optimal pH ranges for PAL enzymes are also compatible with proposed physiological pH of the intestine. The multiple potential sources of PAL include plants such as parsley (*Petroselinum crispum*), eubacteria (*Streptomyces maritimus*), cyanobacteria (*Anabaena variabilis, Nostocpunctiforme*) and yeast (*Rhodoturula glutinis*) or other as yet un-discovered sources.

The selected PAL gene may be codon optimized to allow for successful synthesis (translation) of the enzyme in its probiotic host. This is because each organism has a different codon usage frequency for translating mRNAs into proteins/enzymes, and the PAL gene to be inserted must comply with the host codon usage rather than that of its donor. Each organism typically has multiple codon choices available for each amino acid (AA). Therefore, there are multiple potential DNA sequences that may be used in any particular organism that will result in the same AA sequence while still complying with the probiotic host's codon usage.

Modifications in front of the gene selected for the GMP system may be generated to regulate transcription, translation, and location of the translated PAL enzyme. Additionally, a transcriptional terminator may be added to the end of the gene. Restriction sites may be added as needed throughout the gene. In general, known protocols will be used for DNA amplification, electrophoresis and isolation. In some cases, gel electrophoresis may be conducted in an agarose gel loaded in a "mirror" pattern, such that the same samples are loaded in wells equidistant from an imaginary line running down the center of the gel. After running the gel, the gel will be cut in half along the center of the "mirror". One half will be stained with Ethidium bromide and visualized. The visualized bands will be measured for distance from the load point, and that measurement will be used to cut out the band on the unstained (Ethidium bromide free) half of the gel. This reduces the likelihood of spontaneous mutation during handling.

***Transcription:*** A promoter with constitutive expression may be selected and placed in front of the PAL gene in order to drive transcription of the gene. The promoter may be active *in vivo,* or both *in vivo* and *in vitro.* Several examples of good constitutive promoters in *Lactobacilli* and *Lactococci* include, but are not limited to: *ermB, ldhL, lacA,* and *slp.* It is also possible to place a promoterless PAL gene in tandem behind a gene with a functional promoter as long as the gene with a functional promoter does not contain an intervening termination sequence. If the gene with a promoter does contain a termination sequence, the PAL gene must disrupt the original termination sequence while replicating the termination sequence at the end of PAL.

***Translation:*** Following the promoter there may be a ribosome binding site (RBS) with an appropriate length of spacer prior to the start codon. Although there are some general similarities of RBS sequences and spacer length amongst prokaryotes, each species has its own preferences. For *Lactobacillus* and *Lactococci,* a RBS that is rich in adenine (A) and guanine (G) and is 5-6 bases in length appears to be optimal. The spacer following the RBS and ending at the start codon tends to be between 7 and 13 bases long.

***Enzyme location:*** If it becomes desirable to secrete PAL to the cell's exterior, a secretion tag from another enzyme may be added. Examples include, but are not limited to, the secretion tags from the genes Lp_0373, *amyL, nlp1, usp45,* and *mub.*

The final gene construct for the GMP system must be introduced into the probiotic in a stable manner. This may be done by placing a PAL gene construct into an existing high retention plasmid or high retention shuttle plasmid (with or without a selection cassette), synthetically creating a high retention plasmid/shuttle plasmid, homologously recombining the PAL gene into the probiotic's chromosome, phage transduction, or usage of a transposable element. A high retention plasmid is designed to be retained in the bacteria for several generations in the absence of selective pressure. If selection is used in the mouse version of this treatment, an antibiotic resistance gene may be used, such as an ampicillin or erythromycin resistance gene. In some embodiments, erythromycin resistance genes C, B, or GT may be used. An *E. coli* origin of replication to the plasmid may be used, such as the origin in pUC19 (originally from pMB1). In a human version of this treatment, and FDA approved selection cassette would be used, as antibiotic resistance is not acceptable in human probiotics.

After transformation of the probiotic with the gene construct, *in vitro* phe metabolism by the GMP can be assessed by testing for an increase in absorbance at a wavelength of 280nm indicating increase in trans-cinnamic acid production.

The genetically modified probiotic "PHEnominal" organism disclosed herein will have the ability to live in the small intestine, where it will break down phenylalanine (phe) prior to its absorption into the blood stream. By preventing excess phenylalanine from entering the blood stream, phenylalanine toxicity will be prevented.

In certain embodiments, the probiotic organism of the present invention will be delivered to an animal or patient as a purified microbe free of any culture media. Purified probioitic organisms may be administered in a pill, powder or drops. These methods of administration have the advantage that a higher quantity of the organism may be delivered than with a culture medium such as yogurt (i.e. the potential quantity of yogurt to be ingested could be much more than would typically be consumed). Moreover, a purified probiotic organism such as the present invention does not present problems with lactose or dairy intolerant patients, or infants who are not ready to eat solid foods. Finally, administration by pill, powder or drops has the advantage that the probiotic organism can be administered with a range of foods, and patients will not develop a dislike of a food eaten every day, as could be the case with probiotic delivered in yogurt.

The appropriate dosage of the GMP system can be calculated by measuring the number of live organisms which must be consumed by an animal to be recovered live from the feces. This number can be combined with survival tests of the probiotic to arrive at an approximate dosage.

The genetically engineered probiotic can be tested as a treatment using an animal model, for example the PAHenu2 mouse on the C57BL6 background with the appropriate controls. Blood collection can be used to assess serum phe throughout the experiment. Tissue collection at the end of experiment will allow for other health assessments.

Shipping costs of the probiotic organism of the present invention in a purified and freeze-dried form will be more cost-effective than shipping of probiotic in a culture medium, such as yogurt, which may need to be refrigerated, and which may weigh significantly more. Moreover, the administration of purified probiotic in pill, powder, or drop form, as in the present invention, may allow a greater deree of dosage tailoring from patient to patient and possibly day to day for the same patient than probiotic delivered in culture media such as yogurt.

Patients with PKU have typically not eaten dairy products due to their high phe content. The sudden addition of regular and potentially high amounts of dairy could cause side effects in these patients, which would again favor the administration of purified microbe in pill, powder or drop form, as described herein.

Probiotics are comparatively inexpensive to produce. Because the proposed genetically modified probiotic would be classified as a "drug", it may be more likely to be accepted by insurance companies as a treatment they cover (when compared to synthetic foods). Using a phenylalanine degrading probiotic will likely result in higher treatment compliance, as it will allow patients to maintain a more normal diet and life style and not one in which they would have to ingest distasteful foods. As treatment with "PHEmoninal" would require oral administration, it would not require injections as with the PEG-PAL treatment (see above).

### EXAMPLE 1

### Construction of a GMP System - PHEnominal

The species *Lactobacillus reuteri* is used as the probiotic. Strains of this organism are found in the small intestine of most higher animals including humans and mice. Strains that are human and mouse specific have been identified and studied. For the proof of principal experiments *in-vitro* and the mouse *in-vivo* work, we will use *Lactobacillus reuteri* 100-23C. This strain has no indigenous plasmids and is capable of colonizing the small intestine of *Lactobacillus* free mice. When progressing to human clinical trials, a human specific strain of *L. reuteri* or an alternative probiotic as approved by the FDA will be used.

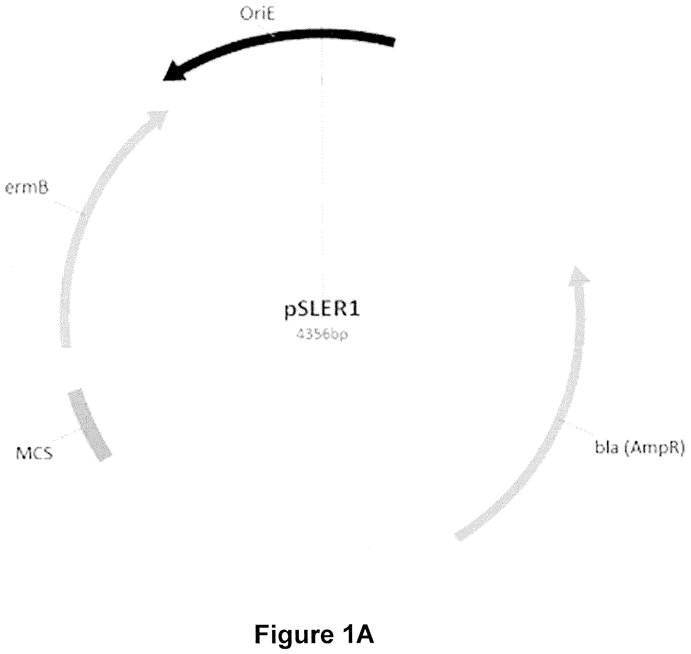

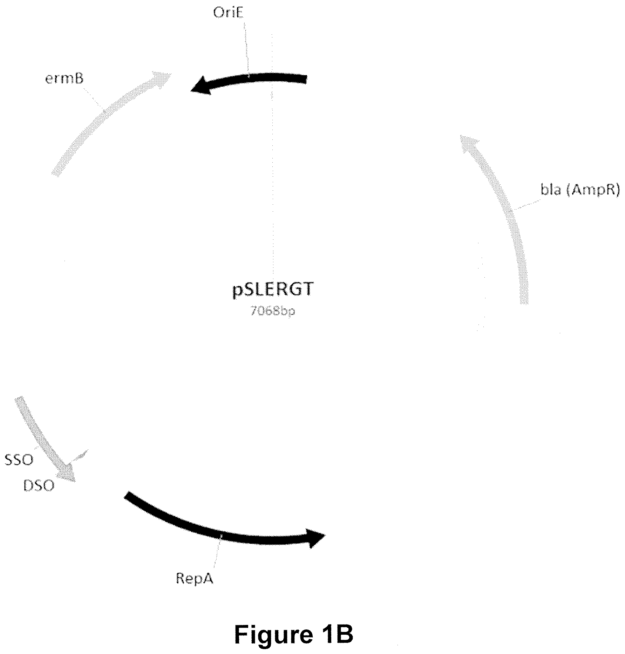

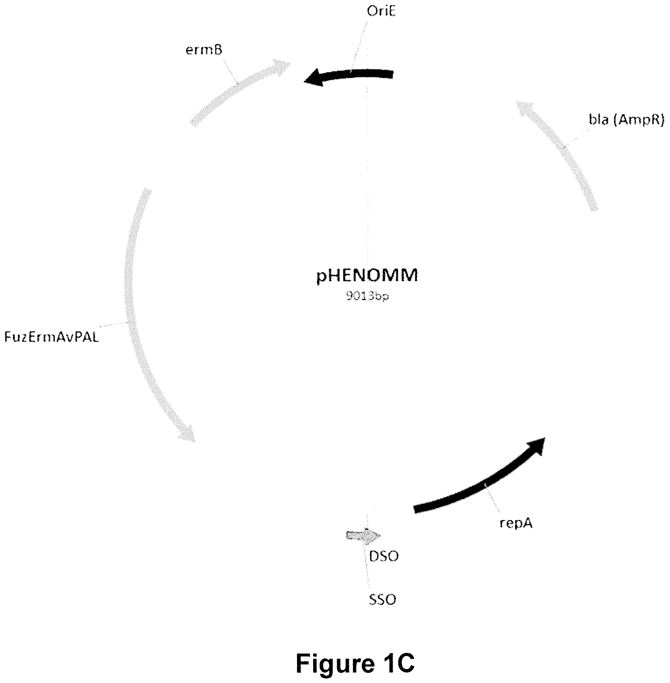

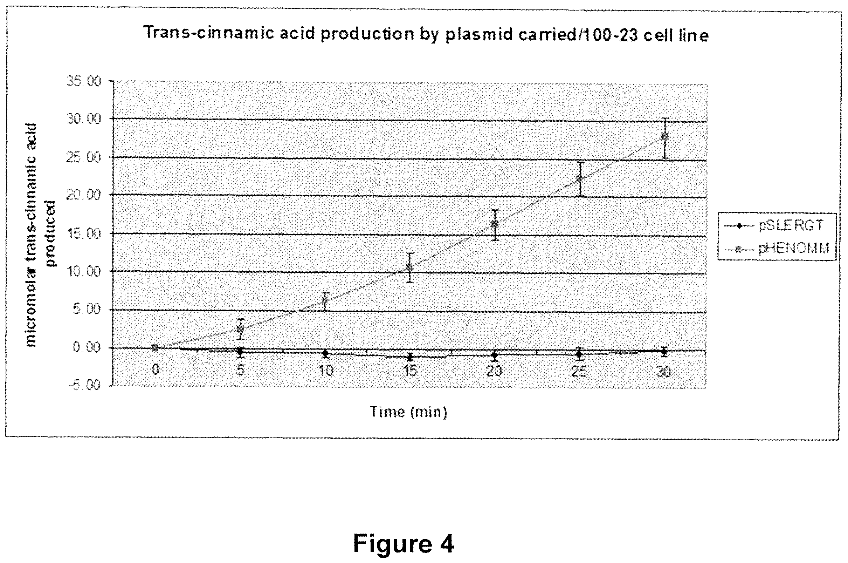

The PAL enzyme to be used is from the cyanobacterium *Anabaena variabilis* ATCC 29413, referred to hereinafter as AvPAL. The sequence for this gene will be codon optimized to match codon usage for the *Lactobacillus reuteri* species as a whole. The AvPAL front sequence (SEQ ID NO: 1) will be placed in front of the codon optimized gene to act as an RBS and spacer sequence. At the end of the gene, the AvPAL end sequence (SEQ ID NO: 2) will be added to act as a transcriptional terminator. This promoterless AvPAL construct (AvPAL plus the above detailed additional sequences) will be synthesized (BioBasic Inc., Ontario, Canada). The complete AvPAL is shown in SEQ ID NO: 3. All sequences not otherwise noted are right facing and in the standard left to right orientation. Plasmids pSLER1, pSLERGT, pHENOMM, and pHENOMM-sec are all left facing and are read right to left. In all plasmids it should be noted that the ermB gene is in the opposite orientation of the plasmid, and this gene is right facing reading left to right (as depicted by plasmid maps in Figures 1A, 1B, and 1C).

A promoter is attached to the AvPAL construct in order to create a functional AvPAL construct. The *ermB* promoter region with 10 additional bases inserted (SEQ ID NO: 4) was selected. This process of fusing the synthesized synthetic *ermB* promoter to AvPAL created the final gene product of FuzErmAvPAL (SEQ ID NO:15) and was performed by BioBasic Inc, Ontario, Canada. Additionally, the secretion tag of Lp_0373 (SEQ ID NO: 5) may be inserted between the ermB promoter and the start codon of the AvPAL gene for secFuzErmAvPAL (SEQ ID NO:6). Once the full construct was created, BioBasic ligated FuzErmAvPAL and secFuzErmAvPAL separately into our synthetically created shuttle vector pSLERGT.

Once FuzErmAvPAL and secFuzErmAvPAL have been synthesised, they must become part of a functional *Lactobacillus reuteri* shuttle plasmid. The shuttle plasmid to be used, pSLERGT (SEQ ID NO: 7), was created specifically for the mouse project by using an antibiotic resistance gene such as erythromycin resistance genes C, B, or GT as the selection cassette, the pGT232 fragment from pNCKH103 (SEQ ID NO: 8) for stability *in-vitro* and *in-vivo* in a *Lactobacillus reuteri* host even in the absence of selection, and *E. coli* origin of replication for shuttle function. The *E. coli* vector pSLER1 (SEQ ID NO: 9) contains the elements *ermB* (erythromycin resistance gene B) and and *E. coli* origin of replication and will be used as the back bone of shuttle vector creation because of these properties. For a human safe plasmid, a selectable gene such as heavy metal resistance or alanine racemase (for complementation) may be used instead of antibiotic resistance. It is also possible in mouse or human safe version to integrate the desired functional AvPAL construct directly into the chromosomal DNA.

To create the desired shuttle vector of pSLERGT, plasmid DNA of pNCKH103 was harvested from 100-23 cells to allow extension PCR of the pGT232 fragment. The reaction used a forward primer for pGT232 amplification (SEQ ID NO: 10) and a reverse primer for pGT232 amplification (SEQ ID NO: 11). The following reaction mixture of New England Biolabs Q5 High Fidelity DNA polymerase was used for a 30µl volume per reaction tube.

| | |
|---|---|
| 10x Q5 polymerase reaction Buffer | 6µl |
| pNCKH103 DNA from L. r. 100-23 | 1.5µl of 15ng/µl total DNA* |
| Forward Primer | 1.5µl |
| Reverse Primer | 1.5µl |
| dNTP 10mM | 2.4µl |
| Water | 18.6µl |
| Q5 DNA polymerase | 0.25µl |

| | |
|---|---|
| * Total DNA, rather than plasmid DNA, is reported due to the dirty quality of DNA extracted from L.r.100-23 cells. | |

The thermocycler conditions used for amplification were

| Step | °C | Time (minutes:seconds) |
|---|---|---|
| 1 | 98.0 | 0:30 |
| 2 | 98.0 | 0:10 |
| 3 | 57.0 | 0:12 |
| 4 | 72.0 | 1:30 |
| 5 | go to step 2, x4 | |
| 6 | 98.0 | 0:10 |
| 7 | 65.0 | 0:12 |
| 8 | 72.0 | 1:30 |
| 9 | Go to step 6, x29 | |
| 10 | 72 | 2:00 |
| 11 | 4 | ∞ |

The resulting PCR products were then run on a 0.8% agarose gel with 0.5% TBE. Gels run to optimize the reaction conditions were run with ethidium bromide incorporated into the gel. Gels for harvesting DNA for plasmid creation were run in the absence of Ethidium Bromide. For harvesting, the gel was loaded such that the first lane was a DNA ladder, and as many remaining lanes as could be filled contained PCR product. Once the Coomassie blue band reached the end of the gel, electrophoresis was stopped. The gel was cut into 2 sections, so that lanes 1 and 2 were together and the remaining lanes in the second portion. The gel segment with lanes 1 and 2 was stained in a 0.5% TBE ethidium bromide bath in a rocker for 20 minutes to visualize the PCR products with a Fotodyne Incorporated Imager and Foto/Analist PC Image software. The desired pGT232 fragment was excised from this stained portion, and revisualized to confirm appropriate cutting. This stained gel was then used as a guide to cut the appropriate bands from the lanes in the unstained portion of the gel. The bands that were never exposed to ethidium bromide or UV light were used in the UltraClean® GelSpin® DNA Extraction Kit (MoBio Laboratories, Inc., Carlsbad, CA). DNA post gel purification was quantitated with a Nanodrop photospectrometer.

The PCR created pGT232 fragment was cut using NcoI FastDigest (Thermoscientific) for one sticky and one blunt end. The *E. coli* vector/backbone for insertion of the PCR product, pSLER1, was cut using FastDigest enzymes NcoI and SmaI resulting in a sticky blunt vector. Reactants for the reactions as follows
pGT232fragment digest
4µl of pGT232fragment at 17ng/µl
0.5 µl 10x ThermoScientific FastDigest buffer
0.5 µl Thermoscientific FastDigest NcoI enzyme
pSLER1 digest
1.3 µl pSLER1 at 750ng/µl
14.7µl water
2 µl 10x ThermoScientific FastDigest buffer
1 µl Thermoscientific FastDigest NcoI enzyme
1 µl Thermoscientific FastDigest SmaI enzyme

The reactions were incubated in a 37°C water bath for 1.5 hours.

Small DNA fragments created by the digests and the enzymes used were removed from the desired DNA using the solutions protocol of the UltraClean® GelSpin® DNA Extraction Kit (MoBio Laboratories, Inc., Carlsbad, CA) and eluted into molecular water. The resulting DNA concentration was again determined by Nanodrop.

Ligation of the digested, PCR created pGT232 fragment into the sticky blunt pSLER1 vector was done using the T4 DNA ligase from Thermoscientific per the following reaction.
Ligation of pGT232fragment into pSLER1 for creation of pSLERGT
2µl 10x T4 DNA ligase Buffer by ThermoScientific
2µl 50% PEG 4000
1 µl pSLER1 digested at 21ng/µl
5.5µl pGT232 fragment digested at 6ng/µl
8.5µl water

Ligation reaction was at room temperature for 60 min. 5 µl of the ligation reaction was added to 50µl of chemically competent Top10 DH5α *E. coli* from Life Technologies for transformation. Transformation was performed as described by the manufacterer. Transformation reaction was plated onto LB agar containing 50µg/mL of ampicillin.

Colonies from the plates were sub-cultured in TB dry containing 300µg/mL of erythromycin. These liquid cultures were used for the UltraClean 6 minute mini plasmid prep kit (MoBio). Plasmid samples were digested with ThermoScientific FastDigest NcoI and SalI, and the resulting digests were run on a 0.8% agarose 0.5% TBE with ethidium bromide gel to detect clones containing a plasmid with the pGT232 fragment insert.

The clone with successful insertion, the desired pSLERGT, was sent to BioBasic for sequencing. Once the sequencing verified the insert contained no mutations to alter function of the pGT232 fragment (SEQ ID NO:12), pSLERGT was transformed into 100-23C cells to verify functionality of the pGT232 segment in-vitro. pSLERGT successfully transformed 100-23 cells in addition to the *E. coli,* proving it a functional shuttle vector.

The reactants and conditions for electotrotransformation of the 100-23C cells are as described in M. A. McCONNELL et al., "Transfer of Plasmid pAMβ1 Between Members of the Normal Microflora Inhabiting the Murine Digestive Tract and Modification of the Plasmid in a Lactobacillus reuteri Host."

Briefly, a Gene Pulser™' apparatus (Bio-Rad Laboratories, Richmond,CA) will be used for all electroporation experiments described in this study. Recipient cells from overnight cultures at 37°C in MRS broth will be used to inoculate MRS broth to an optical density of 0.06 at 600 nm (OD). Cultures will be incubated at 37°C until an OD 600 of 0.8-1.0 is attained. The cells will be harvested by centrifugation, washed twice in electroporation buffer, and then resuspended in electroporation buffer at 1/20th of the original culture volume. Electroporation buffer consists of 952mM sucrose-3.5mM MgCl₂ at pH 7.2 that has been filter sterilized.

Plasmid DNA, ≥ 1µg, will be added to 0.4ml of cells. This mixture is then placed in chilled sterile electroporation cuvettes (0.2cm inter-electrode gap) and held on ice for 5 min. Following the application of a high-voltage electric pulse, voltage of 12,500 V/cm, 200ohm parallel resistance, 25µFD capacitance, the DNA-cell mixture is added to 10mL of pre-warmed non-selective media for a 3 hour recovery period at 37°C. After recovery, cells are harvested by centrifugation and resuspended in 1mL of media for plating. Approximately 50ul of this solution will be plated onto selective media with 5µg/mL of antibiotic. Diluted aliquots will also be plated on media without antibiotic to ensure cells remained viable throughout the procedure regardless of their plasmid uptake. After incubation of the plates in an anaerobe jar at 37°C for approximately 40h, colonies will be counted/utilized.

Colonies that grow will be positive for the plasmid, and these colonies will be grown in an MRS broth culture in the anaerobe jar with its gas pack components at 37 °C over night. Growth from these MRS overnight cultures will be our stock culture, and as such they will be prepared by using a 1:1 ratio of culture to 10% sterile skim milk and saved by freezing at -80 °C.

The final shuttle vector pSLERGT was sent to BioBasic Inc. as mentioned above for ligation of the final FuzErmAvPAL or secFuzErmAvPAL into pSLERGT to create the therapeutic test vectors pHENOMM (SEQ ID NO:13) and pHENOMM-sec (SEQ ID NO:14) respectively.

The resulting plasmid (pHENOMM or pHENOMH) carried by *L. reuteri* are considered an embodiment of the present invention. Probiotic carrying pHENOMM or pHENOMH will be called PHEnominal. Strain specificity of the probiotic and plasmid used denotes its human or mouse usage. Stock vials of the appropriate strain will be thawed and cultured in MRS broth and anaerobic conditions at 37 °C overnight for *in-vitro* and *in-vivo* experimentation. A single vial of stock culture may be used for a long period of study by subculturing the most recent growth of PHEnominal.

### EXAMPLE 2

### Administration and use of the oral PHEnominal to treat PKU

In certain embodiments, the genetically engineered probiotic to treat PKU described herein may be orally administered with food in mice and humans. Survival of the genetically modified probiotic to treat PKU ("PHEnominal") will be greater if it is ingested with food and this will be enhanced further if dairy is ingested concomitant with food. Modes of administration of the oral treatment are varied and may be in pill form, or powder/liquid form. The most appropriate form would be selected for the age and life stage of the patient (e.g. toddlers and infants often resist swallowing pills).

A patient may be provided with access to a phenylalanine monitor to determine levels of phe in the blood. As an essential amino acid, the patient must ensure they are not taking too much of the genetically engineered probiotic. Such phenylalanine monitors for home use are currently in clinical trials, though more expensive versions already exist (approximately $40,000.00 for the currently available machine).

### EXAMPLE 2

At this time, testing of mouse PHEnominal has been performed to demsonstrate functional AvPAL enzyme within the cells. Lysate from 100-23 cells carrying pHENOMM (bearing the FuzErmAvPAL gene, non-secreted) plasmid contain far higher production of trans-cinnamic acid than lysate from cells carrying the empty vector pSLERGT. A representative set of results are seen in Figure 2, and the data retains this trend over multiple experimental replicates. Values depicted indicate the amount of increase in absorbance readings at 280nm, the absorbance point for trans-cinnamic acid/trans-cinnamate, in lysates given phosphate buffered saline with phenylalanine over the amount produced by cells only given phosphate buffered saline alone. Subtracting the values for phosphate buffered saline alone from values measured in the presence of phenylalanine accounts for any increase to the absorbance created by other reactions. These results demonstrate *Lactobacillus reuteri* cells are capable of surviving while producing functional AvPAL enzyme.

### REFERENCES CITED

The following references, to the extent that they provide exemplary procedural or other details supplementary to those set forth herein, are specifically incorporated herein by reference.

### NON-PATENT LITERATURE

Bourget, L, Chang, T. Phenylalanine ammonia-lyase immobilized in semipermeable mircrocapsules for enzyme replacement in phenylketonuria. Federation of European Biochemical Societies, 1985 vol 180 number 1 pp 5-9.
Bourget L, Chang T M S. Biochim Biophys Acta. 1986;883:432-438
Safos S, Chang T M S. Artif Cells Blood Substit Immobil Biotechnol. 1995;23:681-692
Sarkissian C N, Lee K C, Danagher P, Leung R, Fuller M A, Scriver C R. Am JHum Genet Suppl. 1996;59:1183. (abstr.).
Sarkissian C N, Shao Z, Blain F, Peevers R, Su H, Fuller M A, Scriver C R. Am J Hum Genet Suppl. 1997;61:182. (abstr.).
Liu et al. Study on a Novel Strategy to Treatment of Phenylketonuria. Artif cells, Blood Substit Immobil Biotechnol, 2002, 30(4), 243-257. http://www.pkunsw.org.au/research-blog-full-article
M. A. McCONNELL et al., "Transfer of Plasmid pAMβ1 Between Members of the Normal Microflora Inhabiting the Murine Digestive Tract and Modification of the Plasmid in a Lactobacillus reuteri Host"

### CLAUSES:

1. A composition for treating phenylketonuria, comprising:
   a) a probiotic bacterium; and
   b) a gene capable of expressing a functional enzyme for phenylalanine degradation by the probiotic bacterium.
2. The composition of clause 1, wherein the probiotic bacterium is selected from the *Lactobacilli* and *Lactococci* genera.
3. The composition of clause 1, wherein the probiotic bacterium is *Lactobacillus reuteri.*
4. The composition of clause 1, wherein the gene capable of expressing a functional enzyme for phenylalanine degradation is selected from the enzyme family of phenylalanine ammonia-lyases.
5. The composition of clause 4, wherein the gene capable of expressing a functional enzyme for phenylalanine degradation is derived from parsley (*Petroselinum crispum),* eubacteria (*Streptomyces maritimus),* cyanobacteria (*Anabaena variabilis, Nostoc puhctiforme*) or yeast (*Rhodoturula glutihis*).
6. The composition of clause 1, wherein the gene capable of expressing a functional enzyme for phenylalanine degradation is cyanobacterium *Anabaena variabilis* ATCC 29413.
7. The composition of clause 1, wherein the gene capable of expressing a functional enzyme for phenylalanine degradation is codon-optimzed.
8. The composition of clause 1, wherein the gene capable of expressing a functional enzyme for phenylalanine degradation includes a promoter.
9. The composition of clause 8, wherein the promoter exhibits constitutive expression.
10. The composition of clause 1, wherein the gene capable of expressing a functional enzyme for phenylalanine degradation includes a ribosome binding site.
11. The composition of clause 1, wherein the gene capable of expressing a functional enzyme for phenylalanine degradation includes a secretion sequence.
12. The composition of clause 1, wherein the gene is capable of expressing a functional enzyme for phenylalanine degradation is located in a high retention plasmid within the probiotic.
13. The composition of clause 1, wherein the gene capable of expressing functional enzyme for phenylalanine degradation comprises SEQ ID NO:3, or a functional variant thereof.
14. The composition of clause 1, wherein the gene capable of expressing functional enzyme for phenylalanine degradation comprises SEQ ID NO:13, or a functional variant thereof.
15. The composition of clause 1, wherein the gene capable of expressing functional enzyme for phenylalanine degradation comprises SEQ ID NO:14, or a functional variant thereof.
16. A method for treating phenylketonuria, comprising the step of:
   administering the composition of clause 1 to a patient.
17. The method of clause 16, wherein administration is oral.

## Claims

1. A composition, comprising:
a) a probiotic bacterium, wherein the probiotic bacterium is selected from the *Lactobacillus* genera; and
b) a recombinant plasmid vector comprising a nucleotide sequence of SEQ ID NO: 13 or SEQ ID NO: 14 encoding a phenylalanine ammonia-lyase (PAL), wherein the recombinant plasmid vector is retained within the probiotic bacterium.

2. The composition of claim 1, wherein the probiotic bacterium is a *Lactobacillus* strain capable of surviving in human intestine.

3. The composition of claim 1, wherein the nucleotide sequence of SEQ ID NO:13 or SEQ ID NO:14 encoding a phenylalanine ammonia-lyase (PAL) is cyanobacterium *Anabaena variabilis* ATCC 29413.

4. The composition of claim 1, wherein the nucleotide sequence of SEQ ID NO:13 or SEQ ID NO:14 encoding a phenylalanine ammonia-lyase (PAL) is codon-optimzed.

5. The composition of claim 1, wherein the nucleotide sequence of SEQ ID NO:13 or SEQ ID NO:14 encoding a phenylalanine ammonia-lyase (PAL) includes a promoter.

6. The composition of claim 5, wherein the promoter exhibits constitutive expression.

7. The composition of claim 1, wherein the nucleotide sequence of SEQ ID NO:13 or SEQ ID NO:14 encoding a phenylalanine ammonia-lyase (PAL) includes a secretion sequence.

8. A method for administering a composition for treating phenylketonuria, comprising the step of:
administering the composition of claim 1 to a patient.

9. The method of claim 8, wherein administration is oral.
